(19) Europäisches Patentamt
European Patent Office
Office européen des brevets

(11) EP 0 935 457 B1

(12) **EUROPÄISCHE PATENTSCHRIFT**

(45) Veröffentlichungstag und Bekanntmachung des Hinweises auf die Patenterteilung:
**07.01.2004 Patentblatt 2004/02**

(21) Anmeldenummer: **96934737.6**

(22) Anmeldetag: **17.10.1996**

(51) Int Cl.[7]: **A61K 9/127**

(86) Internationale Anmeldenummer:
**PCT/EP1996/004526**

(87) Internationale Veröffentlichungsnummer:
**WO 1998/017255 (30.04.1998 Gazette 1998/17)**

(54) **PRÄPARAT ZUM WIRKSTOFFTRANSPORT DURCH BARRIEREN**

PREPARATION FOR THE TRANSPORT OF AN ACTIVE SUBSTANCE ACROSS BARRIERS

PREPARATION POUR LE TRANSPORT DE MATIERE ACTIVE A TRAVERS DES BARRIERES

(84) Benannte Vertragsstaaten:
**AT BE CH DE DK ES FI FR GB GR IE IT LI LU MC NL PT SE**

(43) Veröffentlichungstag der Anmeldung:
**18.08.1999 Patentblatt 1999/33**

(73) Patentinhaber: **IDEA AG**
**80807 Munich (DE)**

(72) Erfinder: **Cevc, Gregor**
**85551 Heimstetten (DE)**

(74) Vertreter: **Maiwald, Walter, Dr. Dipl.-Chem.**
**Maiwald Patentanwalts GmbH**
**Elisenhof**
**Elisenstrasse 3**
**80335 München (DE)**

(56) Entgegenhaltungen:
**EP-A- 0 475 160**
**EP-A- 0 704 206**
**EP-A- 0 707 847**
**DE-C- 4 447 287**
**US-A- 4 921 706**

- **JOURNAL OF LIPOSOME RESEARCH, Bd. 2, Nr. 3, 1992, NEW YORK (US), Seiten 355-368, XP000303898 G. BLUME ET AL.: "drug-carrier and stability properties of the long-lived lipid vesicles, cryptosomes, in vitro and in vivo"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung von Präparaten zur nichtinvasiven Applikation von Wirkstoffen in Form kleinster in einem flüssigen Medium suspendierbarer Flüssigkeitströpfchen mit einer membranartigen Hülle aus einer oder wenigen Molekülagen, die einen Wirkstoff umfassen und insbesondere zum Transport des Wirkstoffes durch Barrieren, beispielsweise natürliche Permeabilitätsbarrieren und Konstriktionen in Häuten, Schleimhäuten, Organen und dergleichen, geeignet sind.

Außerdem betrifft die Erfindung ein Verfahren zur Herstellung solcher Präparate, insbesondere zur nichtinvasiven Verabreichung von Wirkstoffen.

Die Applikation von Wirkstoffen wird häufig durch natürliche Barrieren wie Häute eingeschränkt, die ein ausreichendes Einbringen von Wirkstoffen verhindern, da sie für Wirkstoffe zu wenig durchlässig sind. Aufgrund der Permeationsbarriere der Haut müssen z.B. die meisten gängigen Therapeutika entweder peroral oder parenteral (i.v., i. m., i.p.) verabreicht werden. Intrapulmonale und intranasale Anwendungen von Aerosolen, der Einsatz von Rektalzäpfchen, die Applikation von Schleimhautgelen, occularen Präparaten usw. lassen sich nur an bestimmten Stellen und nicht mit allen Wirkstoffen realisieren. Das Einbringen von Wirkstoffen in das pflanzliche Gewebe unterliegt aufgrund der kutikulären Wachsschichten noch stärkeren Beschränkungen.

Nichtinvasive Applikationen von Wirkstoffpräparaten, die geeignet sind, solche Permeabilitätsbarrieren zu durchdringen, wären in vielen Fällen vorteilhaft. Bei Mensch und Tier würde beispielsweise eine perkutane Applikation solcher Präparate die verabreichten Wirkstoffe vor der Zersetzung im Gastrointestinaltrakt schützen und gegebenenfalls eine modifizierte Agensverteilung im Körper zur Folge haben; sie kann die Pharmakokinetik der Droge beeinflussen und sowohl häufige, als auch einfache, nichtinvasive Behandlung erlauben (Karzel K., Liedtke, R.K. (1989) Arzneim. Forsch./Drug Res. 39, 1487 - 1491). Bei Pflanzen könnte eine verbesserte Penetration durch oder in die Kuticula die für eine gewünschte Wirkung erforderliche Wirkstoffkonzentration senken und zusätzlich die Umweltbelastung signifikant herabsetzen (Price, C.E. (1981) In: The plant cuticle (D.F. Cutler, K.L. Alvin, C.E. Price, Hrsgb.), Academic, New York, pp 237 - 252).

Bestrebungen, die Hautdurchlässigkeit durch geeignete Maßnahmen zu beeinflussen, sind vielfach besprochen worden (siehe z.B. Karzel und Liedtke, op. cit.). Besonders erwähnenswert sind z.B. Jet-injektion (Siddiqui & Chien (1987) Crit. Rev. Ther. Drug. Carrier. Syst. 3, 195 - 208.), der Einsatz von elektrischen Feldern (Burnette & Ongpipattanakul (1987) J. Pharm. Sci. 76, 765 - 773) oder die Verwendung von chemischen Additiva, wie z.B. von Lösungsmitteln oder Tensiden. Eine lange Liste von Hilfsstoffen, die zwecks Erhöhung der Penetration eines wasserlöslichen Wirkstoffes (Nolaxon) in die Haut getestet wurden, ist z.B. in der Arbeit von Aungst et. al. (1986, Int. J. Pharm. 33, 225 - 234) enthalten.

Das bekannteste Verfahren zur Erhöhung der Wirkstoffpenetration durch die Haut oder Schleimhaut beruht auf der Verwendung von Penetrationsverstärkern. Solche Penetrationsverstärker umfassen nichtionische Stoffe (langkettige Alkohole, Tenside, zwitterionische Phospholipide), anionische Stoffe (besonders Fettsäuren), kationische langkettige Amine, Sulfoxide sowie diverse Aminoderivate; sowie amphothere Glycinate und Betaine. Trotz allem ist jedoch das Problem der Wirkstoffpenetration in die Haut bisher nicht - oder nicht befriedigend - gelöst worden.

Eine Übersicht der Maßnahmen, die zwecks Erhöhung der Wirkstoffpenetration durch die pflanzliche Kuticula eingesetzt werden, ist in der Arbeit von Price (1981, op. cit.) zusammengefaßt.

Die bisher ausschließlich occlusiv verwendeten Penetrationsverstärker erhöhen die Penetrationsfähigkeit an der Permeabilitätsbarriere der Haut- oder Schleimhautoberfläche, indem sie die Fluidität eines Teils der Lipide in dieser Barriere erhöhen. Wenn chemische Penetrationsverstärker verwendet wurden, ist es bisher üblich gewesen, diese dem wirkstoffhaltigen Gemisch einfach hinzuzufügen; lediglich im Falle von menschlicher Haut wurden Additiva manchmal auch vorab, in Form einer organischen Lösung, aufgetragen. Diese Darbringungsform hing mit den bisher untersuchten und diskutierten Wirkungsprinzipien von Additiven zusammen: Im allgemeinen ging man davon aus, daß die verstärkte Agenspenetration einerseits auf der Aufweichung (Fluidisierung) der Haut basiert (Golden et. al., (1987) J. Pharm. Sci. 76, 25 - 28). Diese Hautaufweichung geht in der Regel mit einer Zerstörung der Hautoberfläche und ihren schützenden Barriereeigenschaften einher und ist folglich unerwünscht. Andererseits wurde gezeigt, daß manche Wirkstoffe durch die Haut in Form von niedrigmolekularen Komplexen mit den Zusatzmolekülen permeiren (Green et. al. , (1988) Int. J. Pharm. 48, 103 - 111).

Von diesen Konzepten abweichende Vorschläge, wie die epidermale Anwendung von Lipidsuspensionen, brachten bisher wenig Verbesserung. Solche Suspensionen enthalten typischerweise Vesikel oder O/W- bzw. W/O-Emulgatoren.

Der von mehreren Autoren theoretisch diskutierte perkutane Einsatz von Trägern auf Lipidbasis, den Liposomen (Patel, Bioch. Soc. Trans., 609th Meeting, 13, 513 - 517, 1985, Mezei, M. Top. Pharm. Sci. (Proc. 45th Int. Congr. Pharm. Sci. F.I.P.,) 345 - 58 Elsevier, Amsterdam, 1985) zielte hauptsächlich auf die Beein-flussung der Wirkstoffkinetik. Es war vom Einsatz herkömmlicher Lipidvesikel die Rede, die die Haut nicht oder extrem unvollkommen passieren, wie in dieser Anmeldung gezeigt ist. Der Einsatz von Liposomen, Niosomen oder anderen üblichen Lipidvesikeln ist daher auf äußere Hautschichten beschränkt.

Die japanische Patentanmeldung JP 61/271204 A2 [86/271204] griff die Verwendung von Liposomen durch Verwendung von Hydrochinon-Glucosidal als wirkstoffstabilitätserhöhenden Stoff im ähnlichen Sinne auf.

Als Verbesserung wurde in der WO 87/1938 A1 vorgeschlagen, die wirkstoffbeladenen Lipidvesikel zusammen mit einem Gelbildner in Form von 'transdermal patches' zu verwenden. Die Wirkzeit konnte auf diese Weise verlängert, die Penetrationsfähigkeit des Wirkstoffes jedoch kaum erhöht werden. Durch massiven Einsatz von penetrationsförderndem Polyethylenglycol und Fettsäuren zusammen mit Lipidvesikeln gelang es Gesztes und Mezei (1988, Anesth. Analg. 67, 1079 - 1081) eine lokale Analgesie mit lidocainhaltigen Trägern zu erreichen, allerdings erst nach mehreren Stunden occlusiver Applikation und in geringem Maßstab.

Weiterhin wurden Trägerformulierungen aufgefunden, die für eine Penetration in und durch Permeabilitätsbarrieren geeignet sind. So konnten die Ergebnisse von Gesztes und Mezei durch eine Spezialformulierung, die filtrierte, detergenshaltige Lipidvesikel (Liposomen) mit einem deklarierten optimalen Lipid/Tensid Gehalt von 1-40/1, in der Praxis zumeist um 4/1 aufweisen, erstmalig dramatisch übertroffen werden.

Weiterhin wurde erkannt, daß alle solchen Träger für eine Penetration in und durch die Permeabilitätsbarrieren geeignet sind, die genügend elastisch sind, um durch die Konstriktionen der Barriere, z.B. der Haut, dringen zu können. Dies insbesondere dann, wenn die Träger nach der Applikation selbst einen Gradienten an der Permeabilitätsbarriere aufbauen, da sie in diesem Fall zur spontanen Penetration der Permeabilitätsbarriere tendieren. In den Patentanmeldungen DE 41 07 152 und DE 41 07 153 sind erstmalig Träger, im folgenden als Transfersomen bezeichnet, beschrieben, die zum Wirkstofftransport durch nahezu beliebige Permeationshindernisse tauglich sind.

Transfersomen unterscheiden sich von den bisher für die topische Anwendung beschriebenen Liposomen und von sonstigen verwandten Trägern in mehreren Grundeigenschaften. Transfersomen sind in der Regel viel größer als herkömmliche mizellenartige Trägerformulierungen und unterliegen daher anderen Diffusionsgesetzen. So ist die Permeabilität keine lineare Funktion des Antriebsdruckes, wie bei Liposomen, d.h. bei Transfersomen nimmt die Permeabilität im Gegensatz zu Liposomen oder anderen bekannten ähnlichen Trägersystemen bei steigendem Druck überproportional bzw. nicht linear zu. Ferner können mittels Transfersomen durch Konstriktionen eingebrachte Substanzen im Menschen fast 100% des maximal erreichbaren biologischen oder therapeutischen Potentials entfalten. So erreichen beispielsweise regelmäßig mehr als 50%, häufig mehr als 90%, der perkutan applizierten transfersomal verpackten Wirkstoffe ihren Bestimmungsort im Körper. Diese in der EP 91 114 163 und PCT/EP 91/01596 beschriebenen Transfersomen weisen einen Gehalt einer randaktiven Substanz auf, der bis zu 99 Mol% des Gehaltes und wenigstens 0,1 Mol% dieser Substanz entspricht, durch den der Solubilisierungspunkt der Tröpfchen erreicht wird.

Als entscheidende Bedingung für die gesteigerte Penetrationsfähigkeit der Transfersomen gegenüber Liposomen oder anderen ähnlichen bekannten Trägern wurde dabei der Gehalt an randaktiver Substanz angegeben, der eine optimierte Annäherung an die Solubilisierungsgrenze der Transfersomen bewirkt, (d.h. an einen Gehalt an randaktiver Substanz, der die Transfersomen vollkommen destabilisiert), damit sie genügend elastisch sind, um die Konstriktionen in der Barriere, z.B. in der Haut, durchdringen zu können.

Es wäre nun höchst wünschenswert, bei der Formulierung solch hochgradig permeationsfähiger Präparate nicht an die genannten Gehaltsbereiche gebunden zu sein.

Es ist daher eine Aufgabe der Erfindung, Transfersomen, die entweder keinen Solubilisierungspunkt haben oder weit entfernt vom Solubilisierungspunkt sind, für die Applikation von Wirkstoffen anzugeben, die deren schnellen und wirksamen Transport durch Barrieren und Konstriktionen gestattet.

Aufgabe der Erfindung ist es weiterhin, Transfersomen zum Wirkstofftransport durch menschliche, tierische und pflanzliche Barrieren zur Verfügung zu stellen, die eine verbesserte Verfügbarkeit des Wirkstoffes am Wirkungsort ermöglichen.

Aufgabe der Erfindung ist es weiterhin, ein Verfahren zur Herstellung solcher Transfersomen zum Wirkstofftransport anzugeben.

Zur Lösung dieser Aufgabe dienen die Merkmale der unabhängigen Ansprüche.

Vorteilhafte Ausgestaltungen sind in den Unteransprüchen angegeben.

Überraschenderweise wurde gefunden, daß auch Transfersomenpräparate gebildet werden können, die zur Applikation bzw. zum Transport von wenigstens einem Wirkstoff, insbesondere für medizinische und biologische Zwekke, in und durch natürliche Barrieren und Konstruktionen wie Häute und dergleichen geeignet sind und die Form von in einem flüssigen Medium suspendierbaren Flüssigkeitströpfchen haben, die mit einer membranartigen Hülle aus einer oder wenigen Lagen amphiphiler Trägersubstanz versehen sind, wobei die Trägersubstanz wenigstens zwei (physiko)chemisch verschiedene amphiphile Komponenten umfaßt, die sich in ihrer Löslichkeit im Suspensionsmedium der Transfersomen (üblicherweise Wasser), um einen Faktor von mindestens 10 unterscheiden, wenn deren Gehalt an solubilisierenden Komponenten weniger als 0,1 Mol.-% bezogen auf den Gehalt an diesen Substanzen beträgt, bei dem der Solubilisierungspunkt der umhüllten Tröpfchen erreicht wird oder die amphiphilen Komponenten so ausgewählt sind, daß konzentrationsunabhängig überhaupt keine Solubilisierung der umhüllten Tröpfchen erfolgt.

Die erfindungsgemäßen Präparate, im folgenden wiederum als Transfersomen bezeichnet, können aus beliebigen amphiphilen Komponenten hergestellt werden, die ausreichend unterschiedliche Löslichkeiten aufweisen. Diese Bedingung wird erfüllt, wenn die Löslichkeiten der einzelnen Trägerkomponenten des Transfersoms im Suspensionsmedium sich um mindestens den Faktor $10^1$ (und bis zu $10^7$) unterscheiden. Die Erfüllung dieser Bedingung sorgt dafür, daß die membranartige Hülle der resultierenden Transfersomen unter dem Einfluß eines Gradienten, beispielsweise an einer intakten natürlichen Barriere wie der Haut, eine gesteigerte Deformierbarkeit besitzt. Diese Eigenschaft befähigt die erfindungsgemäßen Transfersomen zur Penetration durch die Konstriktionen in beliebigen Permeabilitätsbarrieren.

Die Fähigkeit der erfindungsgemäßen Präparate, durch Konstriktionen zu permiieren, beträgt mindestens 0,01 Promille, vorzugsweise jedoch mehr als 1 Promille der Permeabilität von kleinen, im wesentlichen ungehindert permeierten Molekülen.

Der Begriff Löslichkeit, wie hier verwendet, bezieht sich nach gegenwärtiger Kenntnis (aber ohne an eine theoretisch-wissenschaftliche Definition gebunden sein zu müssen) auf sogenannte echte Lösungen. Jedenfalls wird bei Erreichen einer jeweiligen Grenzkonzentration eine Löslichkeitsgrenze beobachtet, die durch die Bildung eines Niederschlags, die Bildung von Kristallen, die Bildung von Suspensionen oder durch die Bildung von molekularen Aggregaten wie beispielsweise Micellen definiert ist. Für selbstaggregierende Moleküle entspricht die Löslichkeitsgrenze typischerweise der kritischen Selbstaggregationskonzentration (CAC). Für micellenbildende Moleküle entspricht die Löslichkeitsgrenze typischerweise der kritischen Micellenbildungskonzentration (CMC).

Die erfindungsgemäßen Transfersomen unterscheiden sich erheblich von den bisher beschriebenen Transfersomen. Insbesondere unterscheiden sich die Transfersomen der vorliegenden Anmeldung von bekannten Transfersomen dadurch, daß die Transfersomen aus Kombinationen beliebiger Komponenten, unabhängig von ihrer Solubilisierungsfähigkeit, gebildet werden können.

Außerdem weisen die erfindungsgemäßen Transfersomen eine gegenüber den bekannten Transfersomen (cf. Patentanmeldungen WO 92703122 und EP 475 160) noch verbesserte Stabilität auf, da die Transfersomenzusammensetzung nicht nahe am Solubilisierungspunkt liegt.

Figur 1 zeigt die Abnahme des Permeationswiderstandes an einer Barriere in Abhängigkeit von der Konzentration randaktiver Substanz bezüglich der Annäherung an den Solubilisierungspunkt bei im Stand der Technik beschriebenen Transfersomen, (wobei jedoch dieser Solubilisierungspunkt nicht erreicht wird).

Figur 2 zeigt bei erfindungsgemäßen Transfersomen die Abnahme des Permeationswiderstandes an einer Barriere in Abhängigkeit von der Komponenten-Konzentration bezüglich der Annäherung an einen theoretischen, in der Praxis nicht zu erreichenden Solubilisierungspunkt .

Figur 2 zeigt deutlich, daß für die Komponentensysteme der erfindungsgemäßen Transfersomen keinen Solubilisierungspunkt existiert oder der Solubilisierungspunkt bei Erreichen der maximalen Permeationsfähigkeit noch weit entfernt ist.

Die erfindungsgemäßen Transfersomen öffnen somit einen eleganten, einheitlich und allgemein nützlichen Weg für den Transport von diversen Wirkstoffen in oder durch Permeabilitätsbarrieren. Diese neu entdeckten Wirkstoffträger eignen sich für den Einsatz in Human- und Tiermedizin, Dermatologie, Kosmetik, Biologie, Biotechnologie, Agrartechnologie und in anderen Gebieten.

Ein Transfersom zeichnet sich ferner durch seine Fähigkeit aus, unter der Wirkung eines Gradienten durch und/oder in Permeabilitätsbarrieren zu dringen bzw. diffundieren zu können und dabei Stoffe, insbesondere Wirkstoffe, zu transportieren. Diese Fähigkeit ist insbesondere in der nichtlinearen Permeationsfähigkeit versus Gradient-Kurve leicht zu erkennen und zu quantifizieren.

Ein solches Transfersom setzt sich erfindungsgemäß aus mehreren bis vielen Molekülen zusammen, die physiko-chemisch, physikalisch, thermodynamisch und häufig funktionell eine Einheit bilden. Die optimale Transfersomengröße ist dabei eine Funktion der Barrierecharakteristika. Sie hängt auch von der Polarität (Hydrophilie), Mobilität (Dynamik) und Ladung sowie von der Elastizität der Transfersomen(oberfläche) ab. Ein Transfersom ist vorteilhaft zwischen 10 und 10.000 nm groß.

Für eine dermatologischen Applikationen werden erfindungsgemäß vorzugsweise Transfersomen in der Größenordnung von 50 bis 10.000 nm, häufig von 75 bis 400 nm, besonders häufig von 100 bis 200 nm verwendet.

Für die Applikationen an Pflanzen werden zweckmäßig zumeist relativ kleine Transfersomen, vorwiegend mit einem Durchmesser unter 500 nm eingesetzt.

Der Vesikelradius der Präparat-Tröpfchen (Transfersomen) beträgt ungefähr von 25 bis 500, vorzugsweise von 50 bis 200 und besonders vorzugsweise von 80 bis 180 nm.

Für erfindungsgemäße Transfersomen aus beliebigen Amphiphilen werden bevorzugt eine oder mehrere Komponenten mit einer Wasserlöslichkeit zwischen $10^{-10}$ M und $10^{-6}$ M und ein oder mehrere Komponenten mit einer Wasserlöslichkeit zwischen $10^{-6}$ M und $10^{-3}$ M kombiniert. Alternativ kann man die kombinierbaren amphiphilen Komponenten einander auch über ihre HLB-Werte zuordnen, wobei der Unterschied zwischen den HLB-Werten beider Komponenten vorzugsweise bis 10, häufig zwischen 2 und 7 und besonders häufig 3-5 beträgt.

Die Penetrationsfähigkeit der erfindungsgemäßen Transfersomen kann anhand von Vergleichsmessungen

gegenüber Referenzteilchen oder Molekülen bestimmt werden. Die verwendeten Referenzteilchen sind deutlich kleiner als die Konstriktionen in der Barriere und somit maximal permeationsfähig. Vorzugsweise soll sich die Transfersomenpermeationsrate durch eine Testbarriere ($P_{Transf.}$) von der Permeationsrate der Vergleichsstoffe $P_{Refer}$ (z.B. Wasser), wenn die Barriere selbst der Bestimmungsort ist, um nicht mehr als einen Faktor zwischen $10^{-5}$ und $10^{-3}$ unterscheiden. Wenn ein relativ gleichmäßiger und langsamer Materialtransport durch die Barriere gewünscht ist, soll das angegebene Verhältnis zwischen $10^{-4}$ und 1 liegen. Maximale Penetrationsfähigkeit ist gegeben, wenn das Verhältnis aus $P_{Transf.}/P_{Refer.}$ größer als $10^{-2}$ ist. Diese Angaben beziehen sich auf Transfersomen, die die Konstriktion größenmäßig um mehr als einen Faktor 2 und weniger als 4 überragen. Mit zunehmenden Größenunterschied, Träger/Konstriktion, d. h. bei einem Faktor > 4, können die $P_{Transf.}/P_{Refer.}$ - Werte entsprechend kleiner sein.

Transfersomen gemäß dieser Anmeldungen können aus einer oder mehreren Komponenten bestehen. Am häufigsten verwendet man ein Gemisch von Grundsubstanzen. Geeignete Grundsubstanzen umfassen Lipide und andere Amphiphile, sowie hydrophile Flüssigkeiten; diese können mit den Wirkstoffmolekülen in bestimmten Verhältnissen gemischt werden, die sowohl von der Wahl der Substanzen als auch von ihren absoluten Konzentrationen abhängig sind.

Allgemein weisen die Präparate einen Gehalt von mindestens zwei amphiphilen Komponenten unterschiedlicher Löslichkeit, zur Bildung einer membranartigen Hülle um eine Tröpfchenmenge hydrophiler Flüssigkeit auf, wobei der Wirkstoff in der membranartigen Hülle, beispielsweise einer Doppelmembran und/oder in der hydrophilen Flüssigkeit enthalten ist. Die Wirkstoff-Träger-Assoziierung kann auch wenigstens teilweise erst nach der Bildung von transfersomenartigen Tröpfchen erfolgen.

Wenn die Transfersomen nicht von sich aus ausreichend deformierbar sind und ihre Permeationsfähigkeit durch den Zusatz von randaktiven Stoffen erreicht werden soll, entspricht die Konzentration dieser Stoffe weniger als 0,1 Mol.-% der Menge, die für eine Solubilisierung der Transfersomen erforderlich wäre, oder aber diese Solubilisierung ist im praktisch relevanten Konzentrationsbereich gar nicht erreichbar.

Die erfindungsgemäßen Transfersomen sind zum Wirkstofftransport durch fast beliebige Permeationshindernisse tauglich, z.B. für eine perkutane Medikamentenapplikation. Sie können wasserlösliche, amphiphile oder fettlösliche Agenzien transportieren und erreichen je nach ihrer Zusammensetzung, Applikationsmenge und Form unterschiedliche Penetrationstiefen. Die Spezialeigenschaften, die einen Träger zum Transfersom machen, können sowohl von phospholipidhaltigen Vesikeln, als auch von anderen Amphiphilaggregaten erreicht werden. So kann mittels solcher Transfersomen ein Großteil von Wirkstoffmolekülen nicht nur in die Barriere, z.B. in die Haut, sondern auch durch die Barriere getragen und folglich systemisch aktiv werden. Transfersomen tragen z.B. Polypeptidmoleküle 1.000-fach effizienter durch die Haut als das bisher mit Hilfe von permeatianfördernden strukturlosen Stoffen möglich war.

**DEFINITIONEN:**

**Lipide:**

Ein Lipid im Sinne dieser Erfindung ist jede Substanz, die fettartige oder fettähnliche Eigenschaften besitzt. In der Regel besitzt es einen ausgedehnten apolaren Rest (die Kette, X) und zumeist auch einen wasserlöslichen, polaren, hydrophilen Teil, die Kopfgruppe (Y) und hat die Grundformel 1.

$$X - Y_n \qquad (1)$$

worin n größer oder gleich null ist. Lipide mit n = 0 werden als apolare Lipide bezeichnet, Lipide mit n ≥ 1 werden polare Lipide genannt. In diesem Sinn können alle Amphiphile, wie z.B. Glyceride, Glycerophospholipide, Glycerophosphinolipide, Glycerophosphonolipide, Sulfolipide, Sphingolipide, Isoprenoidlipide, Steroide, Sterine oder Sterole und kohlehydrathaltige Lipide allgemein als Lipide bezeichnet werden.

Ein Phospholipid ist beispielsweise eine Verbindung der Formel 2:

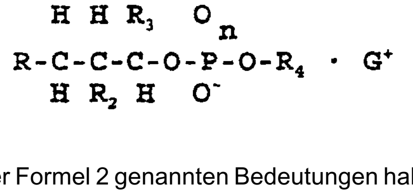

(2)

worin n und $R_4$ die unter Formel 2 genannten Bedeutungen haben, aber $R_1$, $R_2$ nicht Wasserstoff, OH oder kurzkettiger Alkylrest sein kann und $R_3$ meist Wasserstoff oder OH ist. $R_4$ ist außerdem durch Tri-kurzkettiges-Alkylammonio, z.B.

Trimethylammonio, oder Amino substituiertes kurzkettiges Alkyl, z.B. 2-Trimethylammonioethyl (Cholinyl).

Ein Lipid ist vorzugsweise eine Substanz gemäß der Formel 2, worin n = eins, $R_1$ und $R_2$ Hydroxyacyl, $R_3$ Wasserstoff und $R_4$ 2-Trimethyl-ammonioethyl (das letztere entspricht der Phosphatidylcholinkopfgruppe), 2-Dimethylammonioethyl, 2-Methylammonioethyl oder 2-Aminoethyl (entsprechend Phosphatidylethanolaminkopfgruppe) darstellen.

Ein solches Lipid ist z.B. ein natürliches Phosphatidylcholin - veraltet auch Lecithin genannt. Es kann z.B. gewonnen werden aus Ei (reich an Arachidonsäure), Sojabohne (reich an C-18 Ketten), Kokosnuß (reich an gesättigten Ketten), Oliven (reich an einfach ungesättigten Ketten), Safran (Saflor) und Sonnenblumen (reich an n-6 Linoleinsäure), Leinsamen (reich an n-3 Linolensäure), aus Walfett (reich an einfach ungesättigten n-3 Ketten), Nachtkerze oder Primel (reich an n-3 Ketten). Bevorzugte natürliche Phosphatidylethanolamine (veraltet auch Kephaline genannt) stammen häufig aus Ei oder Sojabohnen.

Außerdem sind als Lipide synthetische Phosphatidylcholine ($R_4$ in der Formel 2 entspricht 2-Trimethylammoniumethyl), synthetische Phosphatidylethanolamine ($R_4$ gleich 2-Aminoethyl), synthetische Phosphatidsäuren ($R_4$ ist ein Proton) oder ihre Ester ($R_4$ entspricht z.B. einem kurzkettigen Alkyl, wie Methyl oder Ethyl), synthetische Phosphatidylserine ($R_4$ gleich L- oder D-Serin), oder synthetische Phosphatidyl(poly)alkohole, wie z.B. Phosphatidylinositol, Phosphatidylglycerol ($R_4$ gleicht L- oder D-Glycerol) bevorzugt, worin $R_1$ und $R_2$ identische Acyloxyreste, z.B. Lauroyl, Oleoyl, Linoyl, Linoleoyl oder Arachinoyl bedeuten, z.B. Dilauroyl-, Dimyristoyl-, Dipalmitoyl-, Distearoyl-, Diarachinoyl-, Dioleoyl-, Dilinoyl-, Dilinolenyl-, Dilinoloyl-, Dilinolinoyl, Dilininolenoyl- oder Diarachinoylphosphatidylcholin oder -ethanolamin, oder verschiedene Acylreste, z.B. $R_1$ = Palmitoyl und $R_4$ = Oleoyl, z.B.1-Palmitoyl-2-oleoyl-3-glycerophosphocholin; oder verschiedene Hydroxyacylreste, z.B. $R_1$ = Hydroxypalmitoyl und $R_4$ = Oleoyl usw. sind. Ferner kann $R_1$ Alkenyl und $R_2$ identische (Hydroxy)alkylreste bedeuten, wie z.B. Tetradecylhydroxy oder Hexadecylhydroxy, z.B. in Ditetradecyl- oder Dihexadecylphosphatidylcholin oder - ethanolamin, $R_1$ kann Alkenyl und $R_2$ Hydroxyacyl, z.B. ein Plasmalogen ($R_4$ Trimethylammonioethyl), oder $R_1$ ein Acyl z.B. Lauryl, Myristoyl oder Palmitoyl und $R_2$ Hydroxy sein; so z.B. in natürlichen odersynthetischen Lysophosphatidylcholinen oder Lysophosphatidylglycerolen oder Lysophosphatidylethanolaminen, z.B. 1-Myristoyl- oder 1-Palmitoyllysophosphatidylcholin oder -phosphatidylethanolamin sein; $R_3$ stellt häufig Wasserstoff dar.

Ein geeignetes Lipid im Sinne dieser Erfindung ist auch ein Lipid der Formel 2, worin n = 1 ist, $R_1$ einen Alkenylrest, $R_2$ einen Acyl-amidorest, $R_3$ Wasserstoff und $R_4$ 2-Trimethylammonioethyl (Cholinrest) darstellen. Ein solches Lipid ist unter dem Namen Sphingomyelin bekannt.

Ein geeignetes Lipid ist außerdem ein Lysophosphatidylcholin-Ana-log, z.B. 1-Lauroyl-1,3-propandiol-3-phosphorylcholin, ein Monoglycerid, z.B. Monoolein oder Monomyristin, ein Cerebrosid, Ceramidpolyhexosid, Sulfatid, Sphingoplasmalogen, ein Gangliosid oder ein Glycerid, welches keine freie oder veresterte Phosphoryl- oder Phosphonogruppe oder Phosphinogruppe in 3-Stellung enthält. Ein solches Glycerid ist beispielsweise ein Diacylglycerid oder 1-Al-kenyl-1-hydroxy-2-acylglycerid mit beliebigen Acyl- bzw. Alkenylgruppen, worin die 3-Hydroxygruppe durch einen der genannten Kohlenhydratreste, z.B. einen Galactosylrest, verethert ist, wie z.B. in einem Monogalactosylglycerin.

Lipide mit erwünschten Kopf- oder Kettengruppen-Eigenschaften können auch auf biochemischem Wege, z. B. mittels Phospholipasen (wie Phospholipase A1, A2, B, C und besonders D), Desaturasen, Elongasen, Acyl-Transferasen usw. aus natürlichen oder synthetischen Prekursoren gebildet werden.

Ein geeignetes Lipid ist ferner ein jedes Lipid, welches in biologischen Membranen enthalten und mit Hilfe von apolaren organischen Lösungsmitteln, z.B. Chloroform, extrahierbar ist. Zu solchen Lipiden gehören außer den bereits erwähnten Lipide beispielsweise auch Steroide, z.B. Oestradiol, oder Sterine, z.B. Cholesterin, beta-Sitosterin, Desmosterin, 7-Keto-Cholesterin oder beta-Cholestanol, fettlösliche Vitamine, z.B. Retinoide, Vitamine, z.B. Vitamin A1 oder A2, Vitamin E, Vitamin K, z.B. Vitamin K1 oder K2 oder Vitamin D1 oder D3 usw.

Die weniger lösliche(n) amphiphile(n) Komponente(n) umfaßt bzw. umfassen vorzugsweise ein synthetisches Lipid wie Myristoleoyl-, Palmitoleoyl-, Petroselinyl-, Petroselaidyl-, Oleoyl-, Elaidyl-, cis- bzw. trans-Vaccenoyl-, Linolyl-, Linolenyl-, Linolaidyl-, Octadecatetraenoyl-, Gondoyl-, Eicosaenoyl-, Eicosadienoyl-, Eicosatrienoyl-, Arachidoyl-, cis- bzw. trans-Docosaenoyl-, Doco-sadienoyl-, Docosatrienoyl-, Docosatetraenoyl-, Lauroyl-, Tri-decanoyl-, Myristoyl-, Pentadecanoyl-, Palmitoyl-, Heptadecanoyl-, Stearoyl- bzw. Nonadecanoyl-, Glycerophospholipid bzw. entsprechend kettenverzweigte Derivate oder ein entsprechendes Dialkyl- bzw. Sphingosinderivat, Glykolipid oder anderes Diacyl- bzw. Dialkyl-Lipid.

Die besser lösliche(n) amphiphile(n) Komponente(n) ist häufig von den oben aufgeführten weniger löslichen Komponenten abgeleitet und zur Erhöhung der Löslichkeit mit einem Butanoyl-, Pentanoyl-, Hexanoyl-, Heptanoyl-, Octanoyl-, Nonanoyl-, Decanoyl- oder Undecanoyl-, Substituenten oder mehreren, voneinander unabhängig ausgewählten Substituenten oder mit einem anderen zur Verbesserung der Löslichkeit geeigneten Stoff substituiert und/oder komplexiert, und/oder assoziiert.

Ein geeignetes Lipid ist ferner ein Diacyl- oder Dialkylglycerophosphoethanolaminazopolyethoxylen-Derivat, ein Didecanoylphosphatidylcholin oder ein Diacylphosphooligomaltobionamid.

Als Lipid im Sinne dieser Erfindung gilt auch eine jede andere Substanz (z.B. eine Poly- oder Oligoaminosäure), die eine geringe oder mindestens bereichsweise eine geringe Löslichkeit in polaren Mitteln aufweist.

Alle Tenside ebenso sowie asymmetrische, und daher amphiphile, Moleküle oder Polymere, wie z.B. manche Oligo- und Poly-Kohlenhydrate, Oligo- und Polypeptide, Oligo- und Polynukleotide, viele Alkohole oder Derivate solcher Moleküle gehören in diese Kategorie.

Die Polarität der verwendeten 'Lösungsmittel', Tenside, Lipide oder Wirkstoffe hängt von der effektiven, relativen Hydrophilie/Hydrophobie des jeweiligen Moleküls ab, ist aber auch von der Wahl der sonstigen Systemkomponenten und Randbedingungen im System (Temperatur, Salzgehalt, pH-Wert usw.) abhängig. Funktionelle Gruppen, z. B. Doppelbindungen im hydrophoben Rest, welche den hydrophoben Charakter dieses Restes abschwächen, erhöhen die Polarität; Verlängerung oder raumbeanspruchende Substituenten im hydrophoben Rest, z.B. im aromatischen Rest, erniedrigen die Polarität einer Substanz. Geladene oder stark polare Gruppen in der Kopfgruppe, bei gleichbleibender hydrophoben Kette, tragen normalerweise zu einer höheren Polarität und Löslichkeit der Moleküle bei. Direkte Bindungen zwischen den lipophilen und/oder amphiphilen Systemkomponenten haben eine entgegengesetzte Wirkung.

Als hochpolare Substanzen sind insbesondere alle in der EP-Patentanmeldung 475 160 als randaktiv genannten Verbindungen geeignet. Auf die Offenbarung dieser Patentanmeldung wird hiermit ausdrücklich Bezug genommen.

**WIRKSTOFFE:**

Die erfindungsgemäßen Transfersomen eignen sich zur Applikation unterschiedlichster Wirkstoffe, insbesondere z. B. zu therapeutischen Zwecken. So können erfindungsgemäße Präparate insbesondere alle in der EP-Patentanmeldung 475 160 genannten Wirkstoffe enthalten.

Ferner können erfindungsgemäße Präparate als Wirkstoff ein Adrenocortostaticum, β-Adrenolyticum, Androgen oder Antiandrogen, Antiparasiticum, Anabolicum, Anästheticum oder Analgesicum, Analepticum, Antiallergicum, Antiarrhythmicum, Antiarteroscleroticum, Antiasthmaticum und/oder Bronchospasmolyticum, Antibioticum, Antidepressivum und/oder Antipsychoticum, Antidiabeticum, Antidotum, Antiemeticum, Antiepilepticum, Antifibrinolyticum, Anticonvulsivum, Anticholinergicum, Enzym, Koenzym oder ein entsprechender Inhibitor, ein Antihistaminicum, Antihypertonicum, einen biologischen Aktivitätsinhibitor, ein Antihypotonicum, Antikoagulans, Antimycoticum, Antimyasthenicum, einen Wirkstoff gegen morbus Parkinson oder Alzheimer, ein Antiphlogisticum, Antipyreticum, Antirheumaticum, Antisepticum, Atemanalepticum oder Atemstimulanz, Broncholyticum, Cardiotonicum, Chemotherapeuticum, einen Coronardilatator, ein Cytostaticum, Diureticum, einen Ganglienblocker, ein Glucocorticoid, Grippetherapeuticum, Hämostaticum, Hypnoticum, Immunglobulin bzw. -fragment oder eine andere immunologische bzw. Rezeptor-Substanz, ein bioaktives Kohlehydrat(derivat), ein Kontrazeptivum, ein Migränemittel, ein Mineralcorticoid, einen Morphin-Antagonisten, ein Muskelrelaxans, Narcoticum, Neural- oder CNS-Therapeuticum, ein Nukleotid, oder Polynukleotid, ein Neurolepticum, einen Neurotransmitter oder entsprechenden Antagonisten, ein Peptid (derivat), ein Opthalmicum, (Para)-Sympaticomimeticum oder (Para)-Sympathicolyticum, ein Protein(derivat), ein Psoriasis/Neurodermitismittel, Mydriaticum, Psychostimulanz, Rhinologicum, Schlafmittel oder dessen Antagonisten, ein Sedativum, Spasmolyticum, Tuberlostaticum, Urologicum, einen Vasoconstrictor oder - dilator, ein Virustaticum oder ein Wundenheilmittel oder mehrere solcher Agentien enthalten.

Vorzugsweise ist der Wirkstoff ein nicht-steroidales Antiinflammatoricum, beispielsweise Diclofenac, Ibuprofen oder ein Lithium-, Natrium-, Kalium-, Cäsium-, Rubidium-, Ammonium-, Monomethyl-, Dimethyl-, Trimethyl-Ammonium- oder Ethylammonium-Salz davon.

Außerdem können erfindungsgemäße Präparate als Wirkstoff eine wachstumsbeeinflussende Substanz für Lebewesen, ein Biozid, beispielsweise ein Insektizid, Pestizid, Herbizid, Fungizid, oder einen Lockstoff, insbesondere ein Pheromon aufweisen.

Erfindungsgemäße Präparate können als weniger polare Komponente ein physiologisch verträgliches Lipid, bevorzugt aus der Klasse der Phospholipide, besonders bevorzugt aus der Klasse der Phosphatidylcholine, aufweisen, wobei der Wirkstoff beispielsweise Ibuprophen, Diclofenac oder ein Salz davon, die löslichere Komponente ist, gegebenenfalls mit einem Zusatz von weniger als 10 Gew.-%, bezogen auf die Gesamtzusammensetzung des Präparates einer weiteren löslichen Komponente und wobei die Konzentration der löslicheren Komponente(n) typischerweise zwischen 0,01 Gew.-% und 15 Gew.-%, bevorzugt zwischen 0,1 Gew.-% und 10 Gew.-% und besonders bevorzugt zwischen 0,5 Gew.-% und 3 Gew.-% , und die Gesamtlipidkonzentration zwischen 0,005 Gew.-% und 40 Gew.-%, bevorzugt zwischen 0,5 Gew.-% und 15 Gew.-% und besonders bevorzugt zwischen 1 Gew.-% und 10 Gew.-% beträgt.

Erfindungsgemäße Präparate können zusätzlich Konsistenzbildner, wie Hydrogele, Antioxidantien wie Probucol, Tocopherol, BHT, Ascorbinsäure, Desferroxamin und/oder Stabilisatoren wie Phenol, Cresol, Benzylalkohol und dergleichen umfassen.

Falls nicht anders spezifiziert, können alle angegebenen Substanzen, Tenside, Lipide, Wirkstoffe oder Zusatzstoffe mit einem oder mehreren chiralen Kohlenstoffatomen entweder als racemische Mischungen oder als optisch

reine Enantiomere verwendet werden.

**WIRKPRINZIP:**

Im Falle von Permeations-Barrieren kann der Wirkstofftransport durch solche Transfersomen bewältigt werden, die die folgenden Grundkriterien erfüllen:

- Die Transfersomen sollen einen Gradienten spüren oder aufbauen, der sie in oder über die Barriere treibt, z.B. von der Körperoberfläche in und unter die Haut, von der Blattoberfläche in das Blattinnere, von einer Seite der Barriere zur anderen;

- Der Permeationswiderstand, den die Transfersomen in der Barriere spüren, soll möglichst klein sein im Vergleich zu der treibenden Kraft;

- Die Transfersomen sollen fähig sein, in und/oder durch die Barriere zu permeiren, ohne dabei die eingeschlossenen Wirkstoffe unkontrolliert zu verlieren.

Ferner sollen die Transfersomen vorzugweise eine Kontrolle über die Wirkstoffverteilung, die Wirkstoffeffekte sowie den zeitlichen Wirkungsablauf erlauben. Sie sollen fähig sein, im Bedarfsfall das Material auch in die Tiefe der Barriere und über diese hinweg zu bringen und/oder einen solchen Transport zu katalysieren. Und nicht zuletzt sollen die Transfersomen den Wirkungsbereich und die Wirkungstiefe sowie - in günstigen Fällen - die Art der Zellen, Gewebsteile, Organe, oder Systemabschnitte, die erreicht oder behandelt werden, beeinflussen.

In erster Hinsicht kommen für die biologischen Anwendungen die chemischen Gradienten in Frage. Besonders geeignet sind die physiko-chemischen Gradienten, wie z.B. der (De)Hydratationsdruck (Feuchtigkeitsgradient) oder ein Konzentrationsunterschied zwischen dem Applikations- und Wirkungsort; aber auch elektrische oder magnetische Felder sowie thermische Gradienten sind in dieser Hinsicht interessant. Für technologische Anwendungen sind ferner der applizierte hydrostatische Druck oder ein bestehender Druckunterschied wichtig.

Um die zweite Bedingung zu erfüllen, müssen die Transfersomen auf der mikroskopischen Skala ausreichend 'dünnflüssig' sein, d.h. eine hohe mechanische Elastizität und Verformbarkeit und eine ausreichend niedrige Viskosität; nur dann können sie durch die Konstriktionen innerhalb der Permeabilitätsbarriere gelingen.

Der Permeationswiderstand nimmt verständlicherweise mit der Trägergröße ab. Aber auch die treibende Kraft ist häufig von der Trägergröße abhängig; bei größenunabhängigem Druck nimmt diese Kraft mit der Größe typischerweise ab. Darum ist die Übertragungeffizienz keine einfache Funktion der Größe, sondern weist häufig ein von der Wahl der Träger- und Wirkstoffe abhängiges Maximum auf.

Ferner spielt die Wahl der Trägersubstanzen, Wirkstoffe und Zusatzstoffe, sowie die applizierte Trägermenge oder Konzentration eine Rolle. Niedrige Dosierung führt meistens zu einer oberflächlichen Behandlung: Stoffe, die schlecht wasserlöslich sind, bleiben dabei zumeist in der apolaren Region der Permeabilitätsbarriere (z.B. in den Membranen der Epidermis) hängen; gut lösliche Wirkstoffe, die leicht aus den Trägern diffundieren, können eine andere Verteilung haben als die Träger; für solche Stoffe ist also auch die Durchlässigkeit der Transfersomen-Membran wichtig. Substanzen, die dazu neigen, aus den Trägern in die Barriere überzutreten, führen zu einer örtlich variablen Trägerzusammensetzung, usw. Diese Zusammenhänge sollen vor jeder Applikation überdacht und berücksichtigt werden. Bei der Suche nach Bedingungen, unter denen die einfachen Trägervesikel zu Transfersomen werden, kann die folgende Faustregel verwendet werden.

- Als erstes werden zwei oder mehrere amphiphile Komponenten kombiniert, die sich in ihrer Löslichkeit im vorgesehenen Suspensionsmedium der Transfersomen, üblicherweise Wasser oder ein anderes polares, meist wässriges Medium, um einen Faktor 10 bis $10^7$, vorzugsweise $10^2$ und $10^6$ und besonders bevorzugt zwischen $10^3$ und $10^5$. unterscheiden, wobei die weniger lösliche Komponente eine Löslichkeit von $10^{-10}$ bis $10^{-6}$ und die besser lösliche Komponente eine Löslichkeit im Bereich von $10^{-6}$ bis $10^{-3}$ M aufweist. Die Löslichkeit der entspechenden Komponenten, wenn nicht aus allgemein üblichen Nachschlagewerken bekannt, läßt sich beispielsweise durch herkömmliche Methoden zur Bestimmung der Sättigungsgrenze bestimmen.

- Als nächstes wird die Trägerzusammensetzung bzw. Konzentration der Komponenten im System so angepaßt, daß die Vesikel sowohl eine ausreichende Stabilität als auch eine ausreichende Deformierbarkeit, und daher zweckmäßige Permeationsfähigkeit, aufweisen. Unter Stabilität wird in dieser Anmeldung neben dem mechanischem "Zusammenhalt" auch verstanden, daß sich die Substanz-, insbesondere der Wirkstoffgehalt der Trägerzusammensetzung beim Transport, insbesondere beim Permeationsvorgang, nicht oder nicht wesentlich ändert. Die Position des gesuchten Optimums ist dabei von der Wahl der Komponenten abhängig.

- Abschließend werden die Systemparameter unter Berücksichtigung der angestrebten Applikationsmodi und Ziele nachoptimiert. Für eine rasche Wirkung ist hohe Permeationsfähigkeit erforderlich; für langsame Wirkstoffreisetzung eine allmähliche Barrieren-Penetration und entsprechend eingestellte Membranpermeabilität vorteilhaft; für die Tiefenwirkung ist eine hohe Dosis, für möglichst breite Verteilung eine nicht zu hohe Trägerkonzentration angeraten.

- Der Gehalt an amphiphilen Komponenten wird insbesondere so eingestellt, daß die Fähigkeit des Transfersomen-Präparates, durch Konstriktionen zu permeieren mindestens 0,01 Tausendstel der Permeabilität von kleinen Molekülen (beispielsweise Wasser) beträgt. Die Penetrationsfähigkeit der erfindungsgemäßen Transfersomen kann anhand von Vergleichsmessungen gegenüber Referenzteilchen oder Molekülen bestimmt werden. Die verwendeten Referenzteilchen sind deutlich kleiner als die Konstriktionen in der Barriere und somit maximal permeationsfähig. Vorzugsweise soll die Transfersomenpermeationsrate durch eine Testbarriere ($P_{Transf.)}$ und die Permeationsrate der Vergleichsstoffe ($P_{Refer)}$ (z.B. Wasser), wenn die Barriere selbst der Bestimmungsort ist, sich um nicht mehr als um einen Faktor zwischen $10^{-5}$ und $10^{-3}$ unterscheiden.

In dieser Anmeldung werden relevante Eigenschaften von Transfersomen als Träger für die Lipidvesikel besprochen. Die meisten Beispiele beziehen sich beispielhaft auf die Träger aus Phospholipiden, wobei jedoch die allgemeine Gültigkeit der Schlußfolgerungen nicht auf diese Trägerklasse oder Moleküle beschränkt ist. Die Lipidvesikel-Beispiele illustrieren lediglich die Eigenschaften, die zur Penetration durch die Permeabilitätsbarrieren, wie z.B. Haut, benötigt werden. Dieselben Eigenschaften ermöglichen einen Trägertransport auch durch die tierische oder menschliche Epidermis, Schleimhäute, pflanzliche Kuticula, anorganische Membranen, usw.

Der wahrscheinliche Grund für die spontane Permeation von Transfersomen durch die 'Poren' in der Hornhautzellenschicht ist, daß diese auf einer Seite in einem wässrigen Kompartment, der Subcutis, münden; die Transfersomen werden dabei durch den osmotischen Druck getrieben. Alternativ kann aber zusätzlich ein externer, z.B. hydrostatischer oder elektro-osmotischer Druck appliziert werden.

Je nach Vesikelmenge können nach einer perkutanen Applikation die Lipidvesikel bis in die Subkutis gelangen. Die Wirkstoffe werden dabei, je nach der Größe, Zusammensetung und Formulierung der Träger oder Agenzien, entweder lokal freigesetzt, proximal angereichert, oder aber über die Lymphgefäße bzw. Blutgefäße weitergeleitet und über den Körper verteilt.

Manchmal ist es angebracht, den pH-Wert der Formulierung gleich nach der Herstellung oder unmittelbar vor der Anwendung anzupassen. Eine solche Anpassung soll die Zerstörung der System-komponenten und/oder der Wirkstoffträger unter den anfänglichen pH-Bedingungen verhindern und die physiologische Verträglichkeit der Formulierung gewährleisten. Zur Neutralisierung werden zumeist physiologisch verträgliche Säuren oder Basen bzw. Pufferlösungen mit einem pH-Wert von 3-12, vorzugsweise 5 bis 9, besonders häufig 6-8, je nach dem Zweck und Ort der Applikation, verwendet. Physiologisch verträgliche Säuren sind beispielsweise verdünnte wässrige Mineralsäuren, wie z.B. verdünnte Salzsäure, Schwefelsäure oder Phosphorsäure, oder organische Säuren, z.B. Alkancarbonsäuren, wie Essigsäure. Physiologisch verträgliche Laugen sind z.B. verdünnte Natronlauge, entsprechend ionisierte Phosphorsäure, usw.

Die Herstellungstemperatur wird normalerweise den eingesetzten Substanzen angepaßt und liegt für die wässrige Präparationen üblicherweise zwischen 0 und 95 °C. Vorzugsweise arbeitet man in einem Temperaturbereich von 18-70 °C; besonders bevorzugt für die Lipide mit fluiden Ketten ist der Temperaturbereich zwischen 15 und 55 °C, für die Lipide mit geordneten Ketten zwischen 45 und 60°C. Andere Temperaturbereiche sind für die nichtwässrigen Systeme oder für Präparationen, die Kryo- oder Hitzekonservantien enthalten, bzw. die in situ hergestellt werden, möglich.

Falls die Empfindlichkeit der Systemkomponenten das verlangt, können die Formulierungen kühl (z.B. bei 4°C) gelagert werden. Sie können auch unter Inertgas-, z.B. Stickstoffatmosphäre, hergestellt und aufbewahrt werden. Die Lagerungsdauer kann durch die Verwendung von Substanzen ohne Mehrfachbindungen sowie durch das Eintrocknen und Verwendung von Trockensubstanz, die erst an Ort und Stelle aufgelöst und aufgearbeitet wird, weiter erhöht werden, insbesondere können die transfersomenartigen Tröpfchen kurz vor der Anwendung aus einem Konzentrat oder Lyophilisat zubereitet werden.

In den meisten Fällen findet die Applikation der Träger bei Raumtemperatur statt. Einsätze bei tieferen Temperaturen oder bei höheren Temperaturen mit synthetischen Substanzen noch höhere Temperaturen sind indes durchaus möglich.

Die Herstellung einer Transfersomensuspension kann mittels mechanischer, thermischer, chemischer oder elektrischer Energiezufuhr erfolgen. So kann eine Transfersomenherstellung auf Homogenisierung oder Rühren basieren.

Eine Bildung von transfersomenartigen Tröpfchen kann durch Filtration bewirkt werden. Das dafür verwendbare Filtermaterial sollte eine Porengröße von 0,01 bis 0,8 µm, insbesondere 0,05 bis 0,3 µm und besonders bevorzugt

0,08 bis 0,15 µm aufweisen, wobei gegebenenfalls mehrere Filter hintereinandergeschaltet verwendet werden.

Die Präparate können im voraus oder an Ort und Stelle der Anwendung vorbereitet werden, wie das z.B. in P 40 26 833.0-43 oder anhand mehrerer Beispiele im Handbuch 'Liposomes' (Gregoriadis, G., Hrsg., CRC Press, Boca Raton, Fl., Vols 1-3, 1987) im Buch 'Liposomes as drug carriers' (Gregoriadis, G., Hrsg., John Wiley & Sons, New York, 1988), oder im Labor-atoriumshanduch 'Liposomes. A Practical Approach' (New, R., Oxford-Press, 1989) beschrieben ist. Falls erforderlich, kann eine Wirkstoffsuspension unmittelbar vor dem Gebrauch verdünnt oder aufkonzentriert (z. B. per Ultrazentrifugation oder Ultrafiltration) bzw. mit weiteren Zusatzstoffen vermengt werden. Dabei muß jedoch die Möglichkeit einer Verschiebung des Optimums für die Trägerpermeation ausgeschlossen oder einkalkuliert werden.

Die Transfersomen gemäß dieser Anmeldung sind als Träger von lipophilen Stoffen, z.B. fettlöslichen biologischen Wirkstoffen, Therapeutika und Giften, usw. geeignet; von einem großen praktischen Wert ist auch ihre Anwendung im Zusammenhang mit amphiphilen wasserlöslichen Substanzen, besonders wenn deren Mol-Masse größer als 1000 ist.

Die Transfersomen können ferner zur Stabilisierung von hydrolyseempfindlichen Stoffen beitragen und eine verbesserte Verteilung von Agentien in der Probe und am Ort der Applikation ermöglichen, sowie einen günstigeren zeitlichen Verlauf der Wirkstoffwirkung gewährleisten. Die Grundsubstanz, aus der die Transfersomen bestehen, kann selbst eine vorteilhafte Wirkung haben. Die wichtigste Trägereigenschaft ist jedoch, den Materialtransport in und durch die Permeabilitätsbarriere zu ermöglichen.

Die beschriebenen Formulierungen sind erfindungsgemäß optimiert für die topische Applikation an - oder in der Nähe von - Permeabilitätsbarrieren. Besonders interessant dürfte das Auftragen auf die Haut oder auf die pflanzliche Kuticula sein. (Sie sind aber auch für eine orale (p.o.) oder parenterale (i.v. i.m. oder i.p.) Applikation gut geeignet, besonders wenn die Transfersomenzusammensetzungen so gewählt sind, daß die Verluste am Applikationsort klein sind.). Substanzen bzw. Komponenten, die am Applikationsort bevorzugt abgebaut, besonders stark aufgenommen oder verdünnt werden, sind in letzter Hinsicht in Abhängigkeit vom Einsatzzweck besonders wertvoll.

Im medizinischen Bereich werden bevorzugt bis zu 50, häufig bis zu 10, besonders häufig weniger als 2.5 oder sogar weniger als 1 mg Trägersubstanz pro $cm^2$ Hautfläche aufgetragen; die optimale Menge hängt ab von der Trägerzusammensetzung, angepeilten Wirktiefe und Wirkdauer, sowie von dem Applikationsort. Im agrotechnischen Bereich liegen Applikationsmengen typischerweise niedriger, häufig unter 0.1 g pro $m^2$.

Insbesondere beträgt der Gesamtgehalt an amphiphiler Substanz zur Applikation auf menschlicher und tierischer Haut zwischen 0,01 und 40 Gew.-% des Transfersoms, vorzugsweise zwischen 0,1 und 15 Gew.-% und besonders bevorzugt zwischen 1 und 10 Gew.-%.

Zur Applikation bei Pflanzen beträgt der Gesamtgehalt an amphiphi-ler Substanz 0,000001 bis 10 Gew.-%, vorzugsweise zwischen 0,001 und 1 Gew.-% und besonders bevorzugt zwischen 0,01 und 0,1 Gew.-%.

Je nach der angestrebten Anwendung können die Formulierungen erfindungsgemäß auch geeignete Lösungsmittel bis zu einer Konzentration, die durch die jeweilige physikalische (keine Solubilisierung oder nennenswerte Optimumverschiebung), chemische (keine Beeinträchtigung der Stabilität), oder biologische bzw. physiologische (wenig unerwünschte Nebeneffekte) Verträglichkeit bestimmt wird.

Vorzugsweise kommen dabei unsubstituierte oder substituierte, z.B. halogenierte, aliphatische, cycloaliphatische, aromatische oder aromatisch-aliphatische Kohlenwasserstoffe, z.B. Benzol, Toluol, Methylenchlorid oder Chloroform, Alkohole, z.B. Methanol oder Ethanol, Butanol, Propanol, Pentanol, Hexanol oder Heptanol, Propandiol, Erithritol, Niederalkancarbonsäureester, z.B. Essigsäurealkylester, Ether wie z.B. Diethylether, Dioxan oder Tetra-hydrofuran, oder Mischungen dieser Lösungsmittel, in Frage.

Übersichten der Lipide und Phospholipide, die zusätzlich zu den vorstehend genanntnen für eine Verwendung im Sinne dieser Anmeldung geeignet sind, sind in 'Form and Function of Phospholipids' (Ansell & Hawthorne & Dawson, Verfasser), 'An Introduction to the Chemistry and Biochemistry of Fatty acids and Their Glycerides' von Gunstone und in anderen Übersichtswerken enthalten. Die erwähnten Lipide und Tenside sowie andere, in Frage kommende randaktive Stoffe, und ihre Herstellung, sind bekannt. Ein Überblick der käuflich erhältlichen polaren Lipide, sowie die Warenzeichen, unter denen diese von den Herstellerfirmen vertrieben werden, ist im Jahrbuch 'Mc Cutcheon's, Emulsifiers & Detergents', Manufacturing Confectioner Publishing Co, angegeben. Ein aktuelles Verzeichnis der pharmazeutisch akzeptablen Wirkstoffe ist z. B. dem 'Deutschen Arzneibuch' (und der jeweiligen Jahresausgabe der 'Rote Liste'), ferner aus British Pharmaceutical Codex, European Pharmacopoeia, Farmacopoeia Ufficiale della Republica Italiana, Japanese Pharmacopoeia, Nederlandse Pharmacopoeia, Pharmacopoeia Helvetica, Pharmacopee Francaise, The United States Pharmacopoeia, The United States NF, usw., entnehmbar. Ein ausführliches Verzeichnis der erfindungsgemäß geeigneten Enzyme ist in dem Band 'Enzymes', 3rd Edition (M. Dixon un E.C. Webb, Academic, San Diego, 1979) enthalten, aktuelle Neuentwicklungen sind der Reihe 'Methods in Enzymology' zu entnehmen. Zukkererkennende Proteine, die im Zusammenhang mit dieser Erfindung interessant sind, sind in dem Buch 'The Lectins: Properties, Functions, and Applications in Biology and Medicine' (I.E. Liener, N. Sharon, I.T. Goldstein, Eds. Academic, Orlando, 1986) sowie in aktuellen Fachpublikationen beschrieben; Agrotechnisch interessante Substanzen sind in 'The Pesticide Manual' (C.R. Worthing, S.B. Walker, Eds. British Crop Protection Council, Worcestershier, England, 1986,

z.B. 8th edition) und in 'Wirkstoffe in Pflanzenschutz und Schädlingsbekämpfung', herausgegeben durch den Industrie-Verband Agrar (Frankfurt) angeführt; käuflich erhältliche Antikörper sind in dem Katalog 'Linscott's Directory', die wichtigsten Neuropeptide in 'Brain Peptides' (D.T. Krieger, M.J. Brownstein, J.B. Martin, Eds. John Wiley, New York, 1983), entsprechenden Ergänzungsbänden (z.B. 1987) und anderen Fachpublikationen aufgelistet.

Herstellungstechniken für Liposome, die sich überwiegend auch für die Herstellung von Tranfersomen eignen, sind in 'Liposome Technology' (Gregoriadis, Ed., CRC Press) oder in älteren Nachschlagewerken, z.B. in 'Liposomes in Immunobiology' (Tom & Six, Eds., Elsevier), in 'Liposomes in Biological Systems' (Gregoriadis & Allison, Eds., Willey), in 'Targeting of Drugs' (Gregoriadis & Senior & Trouet, Plenum), usw., sowie in der einschlägigen Patentliteratur beschrieben.

Die Stabilität und Permeationafähigkeit von Transfersomen kann mittels Filtration, ggf. unter Druck, durch ein feinporiges Filter oder durch anderweitige kontrollierte mechanische Aufwirbelung, Scherung oder Zerkleinerung bestimmt werden.

Die folgenden Beispiele veranschaulichen die Erfindung, ohne sie zu beschränken. Temperaturen sind in Grad Celsius, Trägergrößen in Nanometer, Drucke in Pascal und sonstige Größen in üblichen SI Einheiten angegeben.

Verhältnis- und Prozentangaben sind molar, sofern nicht anders angegeben. Meßtemperatur ist ca. 21°C, wenn nicht anders angegeben.

**Beispiele 1 - 4**

**Zusammenfassung:**

| | |
|---|---|
| 0 - 500 mg | Phosphatidylcholin aus Soja-Bohnen CMC ≈ $10^{-7}$ M (ca. 98% PC = SPC) |
| 0 - 500 mg | Distearoylglycerophosphoetha nolamin--triazopolyethoxylen (5000) CMC = $10^{-5}$ M |
| 4.50 ml | Puffer, pH 7,3 |

**Herstellung:**

Es werden Gemische von SPC (angenommene Molmasse: 800 Da) mit zunehmenden Mengen 0, 30 und 40 Mol-% DSPE-PEG (angenommene Molmasse: 5800 Da) und reine DSPE-PEG Liposomen ohne einen Gehalt an SPC hergestellt. Anschließend werden die jeweiligen erhaltenen Gemische in einer Chloroform-Methanol-Lösung gelöst. Danach wird die Lipid-Lösung in ein Rundkolbengefäß übertragen. Nach Entfernen des Lösungsmittels im Rotationsverdampfer bleibt ein dünner Lipidfilm an der Kolbenwand zurück. Dieser Film wird im Vakuum (unter 10 Pa) weitergetrocknet, anschließend durch Pufferzugabe hydratisiert und durch mechanisches Rühren suspendiert. Es wird eine trübe Suspension erhalten, die in der Regel sehr viskos ist. Die Größe der Teilchen in der resultierenden Suspension wird mittels dynamischer Lichtstreuung sowie mittels optischer Mikroskopie bestimmt. Die beobachtete Teilchengröße war in allen Fällen immer größer als 0,5 µm. Anhand der dynamischen Lichtstreuung läßt sich daher für die untersuchten Gemische eine Micellenbildung und folglich auch eine Solubilisierung ausschließen.

Die Liposomen für die Vergleichsversuche werden nach einem analogen Verfahren aus reinem Phosphatidylcholin hergestellt.

**Bestimmung der Träger-Permeationsfähigkeit:**

Eine Trägersuspension wird unter einem gegebenen äußeren Druck durch die Konstriktionen in einer künstlichen Permeationsbarriere getrieben. Die Materialmenge, die pro Zeiteinheit durch die Verengung kommt, wird volumetrisch oder gravimetrisch bestimmt. Aus der Gesamtfläche (Auftragsfläche des Materials), dem (Antriebe-)Druck, der Zeit und der Penetratmenge wird die Permeationsfähigkeit (P) der Suspension im jeweiligen untersuchten System wie folgt berechnet:

$$P = \frac{\text{Penetratmenge}}{\text{Zeit x Fläche x Antriebsdruck}}$$

Die Messung wird unabhängig für mehrere Drucke wiederholt. Aus den Ergebnissen solcher Messungen wird die relative Abhängigkeit der Permeationsfähigkeit, was ein Maß für die Trägerdeformierbarkeit ist, in Abhängigkeit vom mechanischen Streß bzw. Druck berechnet. Der Wert für eine reines SPC enthaltende hydratisierte 1%ige Lösung beträgt bei einem Druck von 0,3 MPa ungefähr >0,01 µl/MPa s $cm^2$ (siehe Figur 3).

Die Permeationsfähigkeitsmessung für solche Versuchsreihen erfolgt bei 62°C, damit sichergestellt ist, daß beide Lipide als fluide Phase vorliegen.

Die Ergebnisse einer solchen Meßreihe für die Beispiele 1 - 4 sind in der Tabelle 1 dargestellt. Tabelle 1 zeigt, daß die Permeationsfähigkeit mit steigendem Antriebsdruck stark, nicht linear ansteigt und bei hohen Tröpfchenbelastungen (0,7 MPa) um mehrere Größenordnungen über dem Wert liegt, der sich bei einer niedrige-ren Belastung (0,3 MPa) ergibt. Ein derartiger ausgeprägter nicht-linearer Zusammenhang gilt jedoch ausschließlich (im Sinne eines Unterscheidungskriteriums) für Transfersomen und nicht für Liposomen. Aus der Figur 3 geht deutlich hervor, daß der Wert der Permeationsfähigkeit für die letztgenannten im Vergleich zu Transfersomen um mehrere Größenordnungen kleiner. Dieser Unterschied der Permeationsfähigkeit zwischen Transfersomen und Liposomen zeigt deutlich die gegenüber Liposomen signifikant gesteigerte Penetrationsfähigkeit.

Tabelle 1

| Proben-Endgröße beschreibung | Druck | Permeations-fähigkeit | Ausgangsgröße | |
|---|---|---|---|---|
| | (MPa) | (µl/MPa s $cm^2$) | (nm) | (nm) |
| SPC/DSPE - PEG | 0,7 | 21,3 | 225,7 | 92,6 |
| 70/30 mol% | 0,6 | 18,7 | | 94,5 |
| 10% Lipidlsg. | 0,5 | 10,9 | | 96,1 |
| rehydratisierte | 0,4 | 2,8 | | 96,1 |
| Probe | 0,3 | 0,007 | | 100,5 |
| SPC/DSPE - PEG | 0,7 | 12,2 | 217,3 | 96,3 |
| 60/40 mol% | 0,6 | 13,2 | | 100,7 |
| 10% Lipidlsg. | 0,5 | 12,2 | | 120 |
| rehydratisierte | 0,4 | 3,39 | | 99,1 |
| Probe | | 0,3 | 0,002 | wenig Filtrat |

## Beispiele 5 - 6

### Zusammensetzung:

| | |
|---|---|
| 410,05 mg, 809,25 mg Bohnen<br>289,95 mg, 190,75 mg<br>7 ml, 10 ml | Phosphatidylcholin aus Soja-(reiner als 95%) CMC ≈ $10^{-7}$ M<br>Didecanoylphosphatidylcholin CMC ≈ $10^{-6}$<br>Puffer, pH 7,3 |

### Herstellung:

Der jeweilige Lipidgehalt wird so gewählt, daß in der endgültigen Formulierung beide Lipid-Komponenten in einem Molverhältnis von 1/1 bzw. 3/1 vorliegen. Die entsprechenden Substanzmengen Phos-phorlipid werden in einem 50 ml Rundkolben eingewogen und in jeweils 1 ml Chloroform/Methanol 1:1 gelöst. Nach Entfernen des Lösungsmittels im Rotationsverdampfer wird, wie in den Beispielen 1-4 zuvor beschrieben, eine Suspension aus dem Film erhalten, die Träger mit einem mittleren Radius von ungefähr 450 nm aufweist.

### Bestimmung der Träger-Permeationsfähigkeit

Die Bestimmung der Träger-Permeationsfähigkeit wird nach dem in den Beispielen 1-4 beschriebenen Verfahren durchgeführt. Die entsprechenden Ergebnisse sind in der Figur 4 dargestellt. Sie zeigen, daß ein Zusatz von Didecanoylphosphatidylcholin die Permeationsfähigkeit der Träger, in Abhängigkeit von der Konzentration, signifikant erhöht, insbesondere bei hohem Druck. Die aus SPC und Didecanoylphosphatidylcholin in einem Molverhältnis von 1/1 gebildeten Träger (davon ausgenommen die Träger mit einem Molverhältnis von 3/1) haben eine signifikant höhere Permeationsfähigkeit als aus reinem SPC gebildete Liposomen.

Die Werte der Permeationsfähigkeit für die gemessenen Träger der Beispiele 5 - 6 sind in der Tabelle 2 zusammengefaßt.

Die reines Didecanoylphosphatidylcholin enthaltende 10%ige Suspension ist milchig trüb. Diese Suspension

enthält Träger mit einem mittleren Durchmesser von 700 ± 150 nm und bildet einen Bodensatz aus. Dieses Verhalten zeigt deutlich, daß das Lipid weder per se noch in Kombination mit SPC im relevanten Konzentrationsbereich solubilisierbar ist.

Tabelle 2

| Probenbeschreibung | Porendurchmesser (nm) | Druck (MPa) | Permeationsfähigkeit |
|---|---|---|---|
| Probe: 3 : 1 | 50 | 0,9 | 0,00039 |
| | 100 | 0,5 | 0,0083 |
| | | 0,6 | 0,021 |
| | | 0,7 | 0,04 |
| | | 0,8 | 0,05 |
| | | 0,9 | 0,066 |
| Probe: 1 : 1 | 50 | 0,9 | 0,16 |
| | 100 | 0,5 | 0,052 |
| | | | 0,021 |
| | | 0,6 | 0,12 |
| | | | 0,17 |
| | | 0,7 | 0,27 |
| | | | 0,22 |
| | | 0,8 | 0,76 |
| | | | 0,69 |
| | | 0,9 | 0,66 |
| | | | 0,60 |

**Beispiel 7**

| | |
|---|---|
| 345,6 mg | Phosphatidylcholin aus Soja-Bohnen (reiner als 95%, PC) CMC = $10^{-7}$ M |
| 154,4 mg | Distearoylphosphomaltobionamid CMC ≤ $10^{-5}$ M |
| 4,5 ml | Puffer, pH 7,3 |

Es wird gemäß dem für die Beispiele 5-6 beschriebenen Verfahren eine Suspension aus SPC/DSPE-Maltobionamid in einem Molverhältnis 3:1 hergestellt. Die resultierenden Träger weisen eine außergewöhnlich gute Permeationsfähigkeit auf. Bei der Bestimmung der Permeationsfähigkeit wird vor und nach jeder Messung die Größe der Träger bestimmt. Die Messungen dienen dem Nachweis, daß zu keinem Zeitpunkt eine Solubilisierung der Träger auftritt.

Die Permeationsfähigkeit der Träger wird bei einem Druck von 0,4 MPa und im Gegensatz zu den Beispielen 5 und 6 bei einer Tempe-ratur von 52°C ermittelt. Bei diesem Druck ist die durch die künstliche Permeationsbarriere beobachtete Trägerpermeation ausreichend gut. Das zugesetzte Lipid (Glyko-Lipid) ist zur Solubilisierung des Phospholipids nicht fähig. Eine Untersuchung der Suspension mittels dynamischer Lichtstreuung sowie mittels optischer Mikroskopie gibt keinen Hinweis auf die Existenz einer solubilisierten (mizellaren) Phase. Die Endgröße der Teilchen beträgt nach der Permeation durch die künstliche Permeabilitätsbarriere in Abhängigkeit vom Antriebsdruck (0,3-0,9 MPa; mit steigendem Druck, Tendenz fallend) zwischen 98 und 81 nm.

Reines Glykolipid geht weder in Lösung noch entsteht eine Mizellensuspension, sondern es bildet sich eine Vesikelsuspension aus. Um das zu belegen, wurde ein Versuch unternommen, womit die osmotische Aktivität von DSPE im wässrigen Medium nachgewiesen werden kann. Hierfür wurde die Lipidsuspension mit Wasser verdünnt. Aufgrund des dadurch entstehenden Konzentrationsgefälles kommt es zum Eintritt von Wasser in die Vesikel. Als unmittelbare Folge nimmt der mittlere Vesikelradius meßbar zu. Dagegen verändern Teilchen ohne Innenvolumen (z. B. Mischmizellen) unter vergleichbaren Versuchsbedingungen ihre Größe nicht.

**Beispiele 8 - 17**

**Zusammensetzung:**

| | |
|---|---|
| 203 - 86,5 µl | Phosphatidylcholin aus Soja-Bohnen (als eine 1:1 Masse / V SPC-Lösung in absolutem Ethanol) CMC (in Wasser) ≈ $10^{-7}$ M |
| 9.04 - 61.4 mg | Diclofenac, Löslichkeit ≤ $10^{-5}$ M |
| 1 ml | Phosphatpuffer (nominal: pH 6,5) |

Die Träger werden nach dem in den Beispielen 1-4 beschriebenen Verfahren als SPC-/Diclofenac-Gemischen in einem Molverhältnis von 4:1 bis 1:4 hergestellt.

Die so erhaltenen Gemische werden einer Ultraschallquelle solange ausgesetzt, bis die Proben makroskopisch klar sind (ungefähr 4 Minuten). Danach werden die Lösungen 15 min bei 15.000 Umdrehungen/min zentrifugiert. Die resultierenden Lösungen 1:1 - 1:4 sind nicht klar (Figur 5), sondern zeigen eine Opaleszenz. Dagegen weisen die Gemische 4:1, 3:1 und 2:1 einen deutlichen Niederschlag auf. Nach 5-minütigen Stehenlassen trüben auch die anderen Suspensionen ein, wobei bei den Gemischen 1:2, 1:3 und 1:4 ein flockiger Niederschlag ausfällt (Tabelle 3). Dieses Verhalten zeigen die Präparate auch nach Einstellen des pH's (mit HCl) auf Werte zwischen pH 7 - pH 7,2.

**Bestimmung der Träger-Permeationsfähigkeit:**

Die Träger-Permeationsfähigkeit, die ein Maß für die Trägerdeformierbarkeit ist, wird wie in den vorangegangenen Beispielen beschrieben, bestimmt. Dabei wird für die Gemische mit 15 mg/ml, 20 mg/ml und 25 mg/ml Diclofenac bei einem Druck von 0,3 MPa (An-triebsdruck) folgende Permeabilitätswerte (P) erhalten: 6x$10^{-11}$ m/Pa/s, $10^{-10}$ m/Pa/s und 2,5x$10^{-10}$ m/Pa/s.

Diese Werte sind mit denen bekannter Transfersomen, die unter ähnlichen Bedingungen gemessen wurden (SPC/NaChol 3/1 M/M; 2 Gew.-%: 3x$10^{-10}$ m/Pa/s), vergleichbar. Das belegt, daß SPC/Diclo-fenac-Gemische geeigneter Zusammensetzung eine sehr hohe Permeationsfähigkeit aufweisen und folglich extrem deformierbar sein müssen, obwohl sie zu keinem Zeit- oder Konzentrationspunkt solubilisierbar sind.

TABELLE 3

| Mit HCl wird der pH auf 7 - 7,2 pH eingestellt und 2 min beschallt. | | |
|---|---|---|
| Nach Beschallen: | 1:1.0 | leicht trüb |
| | 1:1.2 | trüb, flüssig, Kristalle in Lsg. ca. 20 pro Sicht-feld |
| | 1:1.4 | trüb, flüssig, Kristalle in Lsg. ca. 20 pro Sicht-feld |
| | 1:1.6 | trüb, flüssig, Kristalle etwas größer |
| | 1:1.8 | trüb, zähflüssig, Zusammenballung von Kristallen |
| | 1:2.0 | trüb, zähflüssig, sehr viele Kristalle |
| | 1:2.2 | trüb, zähflüssig, sehr viele, sehr große Kristalle |

**Beispiele 18 - 25:**

**Zusammensetzung:**

| | |
|---|---|
| 475 - 325 mg | Phosphatidylcholin aus Soja-Bohnen CMC ≈ $10^{-7}$ M |
| 25 - 175 mg | Ibuprofen, Löslichkeit ≤ 5 x $10^{-5}$ M |
| 5 ml | Puffer, pH 6.5 |

**Herstellung:**

Die Herstellung erfolgt wie in den Beispielen 1-4 beschrieben, mit der Ausnahme, daß der pH-Wert nach Suspensierung des Gemisches durch Zugabe von 10 M NaOH auf pH 7 eingestellt wird. Es werden jeweils 5 ml ibuprofenhaltige Transfersomen mit zunehmender Menge an Ibuprofen und abnehmender Menge an SPC (in 25 mg-Schritten) hergestellt, worin die Gesamtlipidkonzentration 10% beträgt.

**Mikroskopische Kontrolle der erhaltenen Suspensionen:**

Probe 1: keine Kristalle, sehr große Träger;
Probe 2: keine Kristalle, sehr große Träger;
Probe 3: im Hintergrund nur Flimmern;
Probe 4: sehr vereinzelt kleine Kristalle;
Probe 5: keine Kristalle, Tröpfchen;
Probe 6: überwiegend Kristalle;
Probe 7: Tröpfchen, vereinzelt sehr große Kristalle.

**Bestimmung der Träger-Permeationsfähigkeit:**

Die Bestimmung der Träger-Permeationsfähigkeit wird, wie in den vorherigen Beispielen beschrieben, durchgeführt. Die Ergebnisse dieser Messung sind in den Figuren 6 und 7 dargestellt. Die untersuchten Phospholipid-Wirkstoffgemische zeigen durchgehend insbesondere aber im Konzentrationsbereich von 35 mg Ibuprofen/ml und darüber, ein für Transfersomen typisches Verhalten. Die Ibuprofen-Konzentration der Träger bewirkt keine Solubilisierung.

**Vergleichsbeispiele A - E**

Vergleichsbeispiel A (Beispiel 2 aus EP-A 0 211 647)

Zusammensetzung:

| | |
|---|---|
| 120 mg | Dipalmitoylphosphatidylcholin (DPPC) |
| 24 mg | Ölsäure |
| 20 mg | Arginin |
| 60 ml | PBS (eine Tablette in 200 ml dest. Wasser auflösen) |

Es wurden 120,0 mg DPPC und 24,1 mg Ölsäure in ein 100 ml Becherglas eingewogen. Anschließend wurden die beiden Reagenzien vermischt. Eine Phosphatpuffersalz (PBS)-Tablette wurde in 200 ml dest. Wasser vollständig aufgelöst, um einen 10 mM (PBS)-Puffer zu erhalten. Dann wurden 20 mg Arginin in 60 ml PBS, mit einem pH-Wert 7,46 gelöst und dem Lipid-Gemisch zugegeben. Die erhaltene Lösung wurde für eine halbe Stunde auf 40-45°C erhitzt und homogen

Vergleichsbeispiel B (Beispiel 9 aus EP-A 0 280 492)

| | |
|---|---|
| 270 mg | Dipalmitoylphosphatidylcholin (DPPC) |
| 30 mg | DSPC |
| 60 mg | 1-Octadecansulfonsäure (ODS) |

Es wurden 270,05 mg (DPPC), 30,1 mg DSPC und 60,01 mg 1-Octadecansulfonsäure (ODS) in Chloroform/Methanol 1:1 gelöst. Die Probe wurde für zwei Stunden am Rotationsverdampfer bis zur Trockne eingeengt. Anschließend wurde im Vakuum noch für eine Stunde nachgetrocknet. Der Rückstand wurde mit 10 ml PBS rehydriert. Die Mischung wurde auf 60°C erwärmt und homogenisiert. Danach wurde die Probe für 5 Minuten einer Ultraschallquelle ausgesetzt.

Vergleichsbeispiel C (Beispiel 7 aus WO 88/07362):

Zusammensetzung:

| | |
|---|---|
| 400 mg | Setacin F spezial-Paste (Disodiumlaurylsulfo- succinat) |
| 580 mg | hydrogeniertes PC (PHPC) |
| 200 mg | Minoxidil Acetatpuffer pH 5,5 |

Es wurden 400 mg Setacin F Spezial-Paste, 580,03 mg PHPC und 200,03 mg Minoxidil in einem Becherglas eingewogen und mit Chloroform/Methanol 1:1 gelöst und in einen Rundkolben überführt. Das Lipidgemisch wurde am Rotationsverdampfer für ca. 2,5 Stunden eingeengt und anschließend im Vakuum vollständig getrocknet. Dann wurde die Probe bei 50°C im warmen Wasserbad geschwenkt und mit 10 ml Acetatpuffer rehydratisiert. Nach vollständiger Lösung wurde die Lösung für eine Stunde im Wasserbadschüttler stehen gelassen.

Als Antioxidans wurde 1 mg Deferoxamin-Mesylat zugegeben. Dann wurde der pH-Wert der Lösung durch Zugabe von 1 Tropfen 10 mM HCl auf einen pH-Wert von ca. 7,24 eingestellt. Die Lösung ließ sich bei einer Wasserbadtemperatur von 35°C unter Rühren makroskopisch homogenisieren.

Vergleichsbeispiel D (Beispiel 4 aus EP-A 0 220 797)

Zusammensetzung:

| | |
|---|---|
| 400 mg | gereinigtes hydriertes Sojabohnen-Lecithin |
| 40 mg | HCO-60 (Polyoxyethylen hydriertes Rhizinusöl) |
| 100 mg | Vitamin E |
| 9,46 ml | bidest. Wasser |

Es wurden 400,04 mg Phospholipon 90 H (hydriertes Sojabohnenlecithin), 40 mg Eumulgin HRE 60 (Polyoxyethylenhydriertes Rhizinusöl) und 100,11 mg Vitamin E in ein 100 ml Becherglas eingewogen und mit 9,46 ml bidest. Wasser aufgefüllt. Die Probe wurde 45 Minuten, bis fast alles gelöst war, gerührt. Dann wurde die Lipidlösung für 10 Minuten bei 79°C im Ultraschallbad beschallt. Zur vollständigen Lösung wurde die Probe nochmals gerührt und für 10 Minuten bei 56°C im Ultraschallbad beschallt.

Vergleichsbeispiel E (Beispiel 2 aus EP-A 0 102 324)

Zusammensetzung:

| | |
|---|---|
| 300 mg | SPC |
| 150 mg | Octadecyltrimethylammoniumbromid |
| 2550 µl | dest. Wasser |

Es wurden 300 mg SPC und 150 mg Octadecyltrimethylammoniumbromid in ein 100 ml Becherglas eingewogen und mit 1 ml Chloroform/Methanol 1:1 gelöst.

Die Probe wurde im Vakuum bis zur Trocknung eingeengt. Durch Hinzugabe von dest. Wasser wurde eine 1%ige Lösung hergestellt. Die erhaltene Lösung wurde 15 Minuten gerührt.

Die Probenzubereitungen der Vergleichsbeispiele A-E wurden (wenn nicht anders angegeben) den jeweiligen Vorschriften in den genannten Druckschriften entsprechend durchgeführt.

In Figur 8 ist in Form einer Balkengraphik die Permeationsfähigkeit (bei einem konstanten Druck von 0,9 MPa) für die Vergleichsbeispiele A-E und für ein erfindungsgemäßes Ibuprofen-/SPC-Transfersom aufgeführt. Aus der Balkengraphik (Figur 8) geht deutlich hervor, daß die Zusammensetzungen der Vergleichsbeispiele A bis E bei höherem Druck (0,9 MPa) im Vergleich zu erfindungsgemäßen Transfersomen eine signifikant geringere Permeationsfähigkeit

aufweisen.

## Patentansprüche

1. Verwendung von in einem flüssigen Medium suspendierbaren Flüssigkeitströpfchen, die mit einer membranartigen Hülle aus einer oder wenigen Lagen amphiphiler Trägersubstanz versehen sind, wobei die Trägersubstanz wenigstens zwei (physiko)-chemisch verschiedene Komponenten umfaßt, die sich in ihrer Löslichkeit im Suspensionsmedium der Präparate, üblicherweise Wasser, um einen Faktor von mindestens 10 unterscheiden, und der Gehalt solubilisierender Komponenten weniger als 0,1 Mol.-%, bezogen auf den Gehalt an diesen Substanzen, beträgt, bei dem der Solubilisierungspunkt der umhüllten Tröpfchen erreicht wird oder aber dieser Solubilisierungspunkt nicht erreicht werden kann, zur Herstellung eines Präparats zur nichtinvasiven Applikation bzw. zum nichtinvasiven Transport von wenigstens einem Wirkstoff, insbesondere für medizinische oder biologische Zwecke, in und durch Barrieren und Konstriktionen wie Häute und dergleichen.

2. Verwendung nach Anspruch 1,
**dadurch gekennzeichnet, daß** die amphiphilen Komponenten so ausgewählt sind, daß konzentrationsunabhängig keine Solubilisierung erfolgt.

3. Verwendung nach einem der Ansprüche 1 und 2,
**dadurch gekennzeichnet, daß** die Löslichkeit, insbesonders die Wasserlöslichkeit der löslicheren Komponente (n) mindestens $10^{-3}$ bis $10^{-6}$ M und die Löslichkeit, insbesonders die Wasserlöslichkeit der weniger löslichen Komponente(n) mindestens $10^{-6}$ bis $10^{-10}$ M beträgt.

4. Verwendung nach einem der Ansprüche 1 bis 3,
**dadurch gekennzeichnet, daß** der Löslichkeitsunterschied der löslicheren Komponente(n) und der weniger löslichen Komponente(n) ungefähr zwischen 10 und $10^7$, vorzugsweise zwischen $14^2$ und $10^6$ und besonders bevorzugt zwischen $10^3$ und $10^5$ beträgt.

5. Verwendung gemäß einem der Ansprüche 1 bis 4,
**dadurch gekennzeichnet, daß** die Fähigkeit des Präparates, durch Konstriktionen zu permeieren, mindestens 0,01 Promille, vorzugsweise 1 Promille, der Permeabilität von kleinen, im wesentlichen ungehindert permeierenden Molekülen beträgt.

6. Verwendung nach einem der Ansprüche 1 bis 5,
**dadurch gekennzeichnet, daß** das Verhältnis der Permeationsfähigkeit gegenüber Referenzteilchen $P_{(Transf.)}/P_{(Refer)}$, wobei die Referenzteilchen, beispielsweise Wasser, viel kleiner sind als die Konstriktionen in der Barriere, wenn die Barriere selbst der Bestimmungsort ist, zwischen $10^{-5}$ und 1, vorzugsweise zwischen $10^{-4}$ und 1 und besonders bevorzugt zwischen $10^{-2}$ und 1 liegt.

7. Verwendung nach einem der Ansprüche 1 bis 6,
**dadurch gekennzeichnet, daß** das Präparat einen Gehalt von mindestens zwei amphiphilen Komponenten unterschiedlicher Löslichkeit, zur Bildung einer Trägersubstanz und/oder einer membranartigen Hülle um eine Tröpfchenmenge hydrophiler Flüssigkeit umfaßt, worin der Wirkstoff in der Trägersubstanz, in oder an der membranartigen Hülle und/oder in der hydrophilen Flüssigkeit enthalten ist.

8. Verwendung nach einem der Ansprüche 1 bis 7,
**dadurch gekennzeichnet, daß** der Vesikelradius der umhüllten Tröpfchen zwischen ungefähr 25 und ungefähr 500 nm, vorzugsweise zwischen ungefähr 50 und ungefähr 200 nm, besonders bevorzugt zwischen ungefähr 80 und ungefähr 180 nm liegt.

9. Verwendung nach einem der Ansprüche 1 bis 8,
**dadurch gekennzeichnet, daß** die Umhüllung eine Doppelschicht ist.

10. Verwendung nach einem der Ansprüche 1 bis 9,
**dadurch gekennzeichnet, daß** die amphiphile Komponente(n) physiologisch verträgliche Lipide unterschiedlicher Polarität und/oder solche Wirkstoff(e) umfaßt.

**11.** Verwendung nach einem der Ansprüche 1 bis 10,
**dadurch gekennzeichnet, daß** die amphiphile Substanz ein Lipid oder Lipoid biologischer Herkunft oder ein entsprechendes synthetisches Lipid bzw. ein Derivat solcher Lipide ist, insbesondere Diacyl- oder Dialkyl-glycerophosphoethanolaminazopolyethoxylenderivat, Didecanoylphosphatidylcholin, Diacylphosphooligomaltobionamid, ein Glycerid, Glycerophospholipid, Isoprenoidlipid, Sphingolipid, Steroid, Sterin oder Sterol, ein schwefel- oder kohlenhydrathaltiges Lipid, oder aber ein anderes Lipid, das stabile Strukturen, z.B. Doppelschichten bildet, vorzugsweise eine halb protonierte fluide Fettsäure, insbesondere ein Phosphatidylcholin, Phospatidylethanolamin, Phosphatidylglycerol, Phosphatidylinositol, eine Phosphatidsäure, ein Phosphatidylserin, ein Sphingomyelin oder Sphingophospholipid, Glykosphingolipid (z.B. Cerebrosid, Ceramidpolyhexosid, Sulfatid, Sphingoplasmalogen), Gangliosid oder anderes Glykolipid umfaßt, oder ein synthetisches Lipid, vorzugsweise ein Dioleoyl-, Dilinolyl-, Dilinolenyl, Dilinoloyl-, Dilinolinayl-, Diarachinoyl-, Dilauroyl-, Dimyristoyl-, Dilalmitoyl-, Distearoylphospholipid oder ein entsprechendes Dialkyl- bzw. Sphingosinderivat, Glykolipid oder anderes gleich- oder gemischtkettiges Acyl- bzw. Alkyl-Lipid umfaßt.

**12.** Verwendung nach einem der Ansprüche 1 bis 11,
**dadurch gekennzeichnet, daß** die weniger lösliche amphiphile Komponente ein synthetisches Lipid, vorzugsweise Myristoleoyl-, Palmitoleoyl-, Petroselinyl-, Petroselaidyl-, Oleoyl-, Elaidyl-, cis- bzw. trans-Vaccenoyl-, Linolyl-, Linolenyl-, Linolaidyl-, Octadecatetraenoyl-, Gondoyl-, Eicosaenoyl-, Eicosadienoyl-, Eicosatrienoyl-, Arachidoyl-, cis- bzw. trans-Docosaenoyl-, Docosadienoyl-, Docosatrienoyl-, Docosatetraenoyl-, Caproyl-, Lauroyl-, Tridecanoyl-, Myristoyl-, Pentadecanoyl-, Palmitoyl-, Heptadecanoyl-, Stearoyl- bzw. Nonadecanoyl-, glycero-phospholipid bzw. ein entsprechend kettenverzweigtes Derivat oder ein entsprechendes Sphingosinderivat, Glykolipid oder anderes Acyl- bzw. Alkyl-Lipid umfaßt; und die besser lösliche amphiphile Komponente(n) von einer der oben aufgeführten weniger löslichen Komponente abgeleitet ist und zur Erhöhung der Löslichkeit mit einem Butanoyl-, Pentanoyl-, Hexanoyl-, Heptanoyl-, Octanoyl-, Nonanoyl-, Decanoyl-, Dodecan oder Undecanoyl oder einem entsprechend einfach oder mehrfach ungesättigten bzw. kettenverzweigten Substituenten davon oder mehreren unabhängig voneinander ausgewählte Substituenten derivatisiert ist und/oder mit einem anderen zur Verbesserung der Löslichkeit geeigneten Stoff substituiert, komplexiert und/oder assoziiert ist.

**13.** Verwendung nach einem der Ansprüche 1 bis 12,
**dadurch gekennzeichnet, daß** der Gesamtgehalt an amphiphiler Substanz zur Applikation auf menschlicher und tierischer Haut zwischen 0,01 und 40 Gew.-% des Präparates, vorzugsweise zwischen 0,1 und 15 Gew.-% und besonders bevorzugt zwischen 1 und 10 Gew.-% beträgt.

**14.** Verwendung nach einem der Ansprüche 1 bis 13,
**dadurch gekennzeichnet, daß** der Gesamtgehalt an amphiphiler Substanz zur Applikation bei Pflanzen 0,000001 bis 10 Gew.-%, vorzugsweise zwischen 0,001 und 1 Gew.-% und besonders bevorzugt zwischen 0,01 und 0,1 Gew.-% beträgt.

**15.** Verwendung nach einem der Ansprüche 1 bis 14,
**dadurch gekennzeichnet, daß** das Präparat als Wirkstoff ein Adrenocortiostaticum, β-Adrenolyticum, Androgen oder Antiandrogen, Antiparasiticum, Anabolicum, Anästheticum oder Analgesicum, Analepticum, Antiallergicum, Antiarrhythmicum, Antiarterosceroticum, Antiasthmaticum und/oder Bronchospasmolyticum, Antibioticum, Antidepressivum und/oder Antipsychoticum, Antidiabeticum, Antidotum, Antiemeticum, Antiepilepticum, Antifibrinolyticum, Anticonvulsivum, Anticholinergicum, Enzym, Koenzym oder ein entsprechender Inhibitor, ein Antihistaminicum, Antihypertonicum, einen biologischen Aktivitätsinhibitor, ein Antihypotonicum, Antikoagulans, Antimycoticum, Antimyasthenicum, einen Wirkstoff gegen morbus Parkinson oder Alzheimer, ein Antiphlogisticum, Antipyreticum, Antirheumaticum, Antisepticum, Atemanalepticum oder Atemstimulanz, Broncholyticum, Cardiotonicum, Chemotherapeuticum, einen Coronardilatator, ein Cytostaticum, Diureticum, einen Ganglienblocker, ein Glucocorticoid, Grippetherapeuticum, Hämostaticum, Hypnoticum, Immunglobulin bzw. -fragment oder eine andere immunologische bzw. Rezeptor-Substanz, ein bioaktives Kohlehydrat(derivat), ein Kontrazeptivum, ein Migränemittel, ein Mineralcorticoid, einen Morphin-Antagonisten, ein Muskelrelacans, Narcoticum, Neural- oder CNS-Therapeuticum, ein Nukleotid oder ein Polynukleotid, ein Neurolepticum, einen Neurotransmitter oder entsprechenden Antagonisten, ein Peptid (derivat), ein Opthalmicum, (Para)-Sympaticomimeticum oder (Para)-Sympathicolyticum, ein Protein(derivat), ein Psoriasis/Neurodermitismittel, Mydriaticum, Psychostimulanz, Rhinologicum, Schlafmittel oder dessen Antagonisten, ein Sedativum, Spasmolyticum, Tuberlostaticum, Urologicum, einen Vasoconstrictor oder -dilator, ein Virusstaticum oder ein Wundenheilmittel oder mehrere solcher Agentien, insbesondere Diclofenac bzw. Ibuprofen, enthält.

**16.** Verwendung nach einem der Ansprüche 1 bis 15,
**dadurch gekennzeichnet, daß** der Wirkstoff ein nicht-steroidales Antiinflammatoricum, beispielsweise Diclofenac, Ibuprofen oder ein Lithium-, Natrium-, Kalium-, Cäsium-, Rubidium-, Ammonium-, Monomethyl-, Dimethyl-, Trimethylammonium- oder Ethylammonium-Salz davon ist.

**17.** Verwendung nach einem der Ansprüche 1 bis 16,
**dadurch gekennzeichnet, daß** die weniger polare Komponente ein physiologisch verträgliches Lipid, bevorzugt aus der Klasse der Phospholipide, besonders bevorzugt aus der Klasse der Phosphatidylcholine, umfaßt und der Wirkstoff die löslichere Komponente ist, gegebenenfalls mit einem Zusatz von weniger als 10 Gew.-%, bezogen auf die Gesamtzusammensetzung des Präparates einer weiteren löslichen Komponente, die löslichere Komponente ist, wobei die Konzentration der löslicheren Komponente(n) typischerweise zwischen 0,01 Gew.-% und 15 Gew.-%, bevorzugt zwischen 0,1 Gew.-% und 10 Gew.-% und besonders bevorzugt zwischen 0,5 Gew.-% und 3 Gew.-%, und die Gesamtlipidkonzentration zwischen 0,005 Gew.-% und 40 Gew.-%, bevorzugt zwischen 0,5 Gew.-% und 15 Gew.-% und besonders bevorzugt zwischen 1 Gew.-% und 10 Gew.-% beträgt.

**18.** Verwendung nach einem der Ansprüche 1 bis 17,
**dadurch gekennzeichnet, daß** das Präparat Konsistenzbildner, wie Hydrogele, Antioxidantien wie Probucol, Tocopherol, BHT, Ascorbinsäure, Desferroxamin und/oder Stabilisatoren wie Phenol, Cresol, Benzylalkohol und dergleichen umfaßt.

**19.** Verwendung nach einem der Ansprüche 1 bis 18,
**dadurch gekennzeichnet, daß** der Wirkstoff eine wachstumsbeeinflussende Substanz für Lebewesen ist.

**20.** Verwendung nach einem der Ansprüche 1 bis 18,
**dadurch gekennzeichnet, daß** der Wirkstoff biozide Eigenschaften hat, insbesondere ein Insektizid, Pestizid, Herbizid oder Fungizid ist.

**21.** Verwendung nach einem der Ansprüche 1 bis 18,
**dadurch gekennzeichnet, daß** der Wirkstoff ein Lockstoff, insbesondere ein Pheromon ist.

**22.** Verfahren zur Herstellung eines Präparates zur Verwendung gemäß einem der Ansprüche 1 bis 21,
**dadurch gekennzeichnet, daß** wenigstens zwei amphiphile Komponenten ausgewählt werden, die sich in ihrer Löslichkeit im Suspensionsmedium des Präparats, üblicherweise Wasser, um einen Faktor von mindestens 10 unterscheiden, und der Gehalt solubilisierender Komponenten weniger als 0,1 Mol.-%, bezogen auf den Gehalt an diesen Substanzen, beträgt, bei dem der Solubilisierungspunkt der umhüllten Tröpfchen erreicht wird, oder aber dieser Punkt im praktisch relevanten Bereich nicht erreicht werden kann, und der Gehalt an amphiphilen Komponenten so eingestellt wird, daß die Fähigkeit des Präparates, durch die Konstriktionen zu permeieren, mindestens 0,01 Tausendstel der Permeabilität von Wasser, beträgt.

**23.** Verfahren nach Anspruch 22,
**dadurch gekennzeichnet, daß** der Gehalt der amphiphilen Komponenten so eingestellt wird, daß das Verhältnis der Permeationsfähigkeit gegenüber Wasser, wenn die Barriere selbst der Bestimmungsort ist, zwischen $10^{-5}$ und 1, vorzugsweise zwischen $10^{-4}$ und 1, besonders bevorzugt zwischen $10^{-2}$ und 1 beträgt.

**24.** Verfahren nach einem der Ansprüche 22 und 23,
**dadurch gekennzeichnet, daß** man Stabilität und Permeationsfähigkeit mittels Filtration, gegebenenfalls unter Druck, durch ein feinporiges Filter oder durch anderweitige kontrollierte mechanische Aufwirbelung, Scherung oder Zerkleinerung bestimmt.

**25.** Verfahren nach einem der Ansprüche 22 bis 24,
**dadurch gekennzeichnet, daß** das Substanzgemisch, zur Erzeugung eines transfersomenartigen Präparats, einer Filtration, Ultraschallbehandlung, Rühren, Schütteln oder anderen mechanischen Zerteilungseinwirkungen ausgesetzt wird.

**26.** Verfahren nach einem der Ansprüche 22 bis 25,
**dadurch gekennzeichnet, daß** man aus wenigstens zwei amphiphilen Komponenten unterschiedlicher Polarität, wenigstens einer polaren Flüssigkeit und wenigstens einem Wirkstoff transfersomenartige Tröpfchen erzeugt, die das Präparat bilden.

27. Verfahren nach einem der Ansprüche 22 bis 26,
**dadurch gekennzeichnet, daß** man aus wenigstens zwei amphiphilen Komponenten unterschiedlicher Polarität und wenigstens einer polaren Flüssigkeit transfersomenartige Tröpfchen erzeugt, die das Präparat bilden, worin die amphiphile Komponente(n) den Wirkstoff umfaßt oder beinhaltet.

28. Verfahren nach einem der Ansprüche 22 bis 27,
**dadurch gekennzeichnet, daß** man separat jeweils die amphiphilen Komponenten und die hydrophile Substanz mit dem Wirkstoff vermischt und gegebenenfalls zur Lösung bringt, die Gemische bzw. Lösungen dann zu einer Mischung zusammenführt und in dieser durch Zufuhr von insbesondere mechanischer Energie die Tröpfchenbildung bewirkt.

29. Verfahren nach einem der Ansprüche 22 bis 28,
**dadurch gekennzeichnet, daß** die amphiphilen Komponenten entweder als solche oder gelöst in einem physiologisch verträglichen, mit polarer Flüssigkeit(en), insbesondere Wasser mischbaren Lösungsmittel oder Lösungsvermittler mit einer polaren Lösung zusammengegeben werden.

30. Verfahren nach einem der Ansprüche 22 bis 29,
**dadurch gekennzeichnet, daß** die Bildung der umhüllten Tröpfchen durch Einrühren, mittels Verdampfung aus einer Umkehrphase, durch ein Injektions- oder Dialyseverfahren, durch elektrische, thermische oder mechanische Beanspruchung wie Schütteln, Rühren, Homogenisieren, Ultrabeschallen, Reiben, Frieren bzw. Auftauen, Heizen oder Kühlen oder Hoch- oder Niedrigdruck-Filtration herbeigeführt wird.

31. Verfahren nach einem der Ansprüche 22 bis 30,
**dadurch gekennzeichnet, daß** die Bildung der umhüllten Tröpfchen durch Filtration bewirkt wird und das Filtermaterial eine Porengröße von 0,01 bis 0,8 μm, insbesondere 0,05 bis 0,3 μm und besonders bevorzugt 0,08 bis 0,15 μm aufweist, wobei gegebenenfalls mehrere Filter hintereinander geschaltet verwendet werden.

32. Verfahren nach einem der Ansprüche 22 bis 31,
**dadurch gekennzeichnet, daß** die Träger-Wirkstoffassoziation wenigstens teilweise nach der Tröpfchenbildung erfolgt.

33. Verfahren nach einem der Ansprüche 22 bis 32,
**dadurch gekennzeichnet, daß** die umhüllten Tröpfchen kurz vor der Anwendung aus einem Konzentrat oder Lyophilisat zubereitet werden.

**Claims**

1. The use of liquid droplets suspensible in a liquid medium and coated with a membrane-like coating composed of one or several layers of an amphiphilic carrier substance, wherein the carrier substance comprises of at least two (physico)-chemically different components that differ in solubility in the suspension medium of the preparation, usually water, by a factor of at least 10 and wherein the content of solubilizing components is less than 0.1 mol % relative to the content of these substances, whereby the point of solubilization of the coated droplets is reached, or else this point of solubilization cannot be reached, for the manufacture of a preparation for the non-invasive application or for the non-invasive transport of at least one active agent, in particular for medicinal or biological purposes, into and through barriers and constrictions such as are presented by the skin and similar structures.

2. The use according to claim 1, **characterized in that** the amphiphilic components are selected such that independent of concentration no solubilization occurs.

3. The use according to any one of claims 1 and 2, **characterized in that** the solubility, in particular the solubility in water of the soluble component(s) is at least $10^{-3}$ and up to $10^{-6}$ M, and the solubility, in particular the solubility in water, of the less soluble component(s) is at least $10^{-6}$ and up to $10^{-10}$ M.

4. The use according to any one of claims 1 to 3, **characterized in that** the difference in solubility of the soluble component(s) and the less soluble component(s) is approximately from 10 to $10^7$, preferably from $10^2$ to $10^6$ and particularly preferably from $10^3$ to $10^5$.

5. The use according any one of claims 1 to 4, **characterized in that** the capability of the preparation to permeate constricted passages, amounts to at least 0.01 per thousand, preferably 1 per thousand, of the permeability of small, essentially unhindered permeating molecules.

6. The use according any one of claims 1 to 5, **characterized in that** the ratio of the permeation capacity versus the reference particles $P_{(Transf.)}/P_{(Refer)}$, wherein the reference particles, for example water, are much smaller that the constrictions in the barrier, if the barrier itself is the site of measurement, is from $10^{-5}$ to 1, preferably from $10^{-4}$ to 1 and particularly preferably from $10^{-2}$ to 1.

7. The use according any one of claims 1 to 6, **characterized in that** the preparation comprises at least two amphiphilic components of differing solubility for the formation of a carrier substance and/or a membrane-like coating of a droplet amount of hydrophilic liquid, wherein the active agent is contained in the carrier substance, in or on the membrane-like coating and/or in the hydrophilic liquid.

8. The use according any one of claims 1 to 7, **characterized in that** the vesicle radius of the coated droplets is from approximately 25 and to 500 nm, preferably from approximately 50 to approximately 200 nm, particularly preferably from approximately 80 to approximately 180 nm.

9. The use according any one of claims 1 to 8, **characterized in that** the coating forms a double layer.

10. The use according any one of claims 1 to 9, **characterized in that** the amphiphilic component(s) comprises physiologically acceptable lipids of differing polarity and/or such an active agent or active agents, respectively.

11. The use according any one of claims 1 to 10, **characterized in that** the amphiphilic substance comprises a lipid or lipoid of biological origin or a corresponding synthetic lipid or a derivative of such lipids, in particular a diacyl- or dialkyl-glycerophospho-ethanolamine azopolyethoxylene derivative, didecanoylphosphatidylcholine, diacylphospho-oligomaltobioamide, a glyceride, glycerophospholipid, isoprenoid lipid, sphingolipid, steroid, sterin or sterol, a sulphur- or carbohydrate-containing lipid, or another lipid that forms stable structures, for example double layers, preferably a half-protonated liquid fatty acid, in particular a phosphatidylcholine, phosphatidylethanolamine, phosphatidylglycerol, phosphatidylinositol, a phosphatidic acid, a phosphatidylserine, a sphingomyelin or sphingophospholipid, glycosphingolipid (for example cerebroside, ceramidepolyhexoside, sulfatide, sphingoplasmalogen), ganglioside or other glycolipid, or consists of a synthetic lipid, preferably a dioleoyl-, dilinolyl-, dilinolenyl, dilinoloyl-, dilinolinayl-, diarachinoyl-, dilauroyl-, dimyristoyl-, dilalmitoyl-, distearoylphospholipid or a corresponding dialkyl- or sphingosine derivative, glycolipid and other equal or mixed chain acyl- or alkyl-lipids, respectively.

12. The use according any one of claims 1 to 11, **characterized in that** the less soluble amphiphilic component comprises a synthetic lipid, preferably myristoleoyl-, palmitoleoyl-, petroselinyl-, petroselaidyl-, oleoyl-, elaidyl-, *cis*- or *trans*-vaccenoyl-, linolyl-, linolenyl-, linolaidyl-, octadecatetraenoyl-, gondoyl-, eicosaenoyl-, eidosadienoyl-, docosatetraenoyl, caproyl-, lauroyl-, tridecanoyl-, myristoyl-, pendadecanoyl-, palmitoyl-, heptadecanoyl-, stearoyl- or nonadecanoyl-glycerophospholipid or a corresponding branched chain derivative or a corresponding sphingosine derivative, glycolipid or other acyl- or alkyl-lipids; and the more soluble amphiphilic component(s) is/are derived from one of the above less soluble components and a derivative is formed by the use of butanoyl-, pentanoyl, hexanoyl, heptanoyl, octanoyl, nonanoyl, decanoyl, dodecan or undecanoyl or one of the appropriate singly or several times unsaturated or branched-chain substitutes thereof or several substitutes independent of each other and/or substituted, complexed and/or associated with another appropriate solubility-enhancing material.

13. The use according to any one of claims 1 to 12, **characterized in that** the total content of amphiphilic substances for application on human and animal skin amounts to from 0.01 to 40% by weight, preferably from 0.1 to 15% w/w and particularly preferably from 1 to 10% w/w.

14. The use according to any one of claims 1 to 13, **characterized in that** the total amount of amphiphilic substance for application to plants is from 0.000001 to 1% w/w, preferably between 0.001 and 1% w/w and particularly preferably between 0.01 and 0.1% w/w.

15. The use according any one of claims 1 to 14, **characterized in that** it contains, as the active substance, an agent that is adrenocorticostatic, an androgen or antiandrogen, antiparasitic, anabolic, anaesthetic or analgesic, antiallergenic, antiarrhythmic, antiarteriosclerotic, antiasthmatic, and/or bronchospasmolytic, antibiotic, antidepressant and/or antipsychotic, antidiabetic, antidote, antiemetic, antiepileptic, antifibrinolytic, anticonvulsive, anticholinergic,

an enzyme, coenzyme or corresponding inhibitor, an antihistamine, antihypertonic, a biological activity inhibitor, antihypotonic, anticoagulant, antimycotic, antimyasthenic, an agent to treat Parkinson or Alzheimer disease, antiphlogistic, antipyretic, antirheumatic, antiseptic, respiratory analeptic or stimulant, broncholytic, cardiotonic, chemotherapeutic, a coronary dilatator, cytostatic, diuretic, a ganglion blocker, a glucocorticoid, antiinfluenza, haemostatic, hypnotic, an immunoglobulin or fragment or another immunological or receptor substance, a bioactive carbohydrate (derivative), a contraceptive, antimigraine, a mineralcorticoid, a morphine antagonist, muscle relaxant, narcotic, neuro- or CNS-therapy, nucleotide or polynucleotide, neuroleptic, neurotransmitter or corresponding antagonist, a peptide (derivative), an ophthalmologic, (para)-sympathomimetic, a protein (derivative), a psoriasis/neurodermatitis agent, a mydriatic, psychostimulant, rhinologic agent, sleep inducing agent or its antagonist, a sedative, spasmolytic, tuberlostatic, urologic, a vasonconstrictor or -dilator, a virostatic, or a wound-healing agent or several such agents, in particular diclofenac or ibuprofen.

**16.** The use according to one of claims 1 to 15, **characterized in that** the agent is a non-steroidal anti-inflammatory, for example diclofenac, ibuprofen or a lithium-, sodium-, potassium-, caesium-, rubidium-, ammonium-, monomethyl-, dimethyl-, or trimethylammonium or ethylammonium salt thereof.

**17.** The use according to one of claims 1 to 16, **characterized in that** the less polar component comprises a physiologically tolerated lipid, preferably in the class of phospholipids and particularly preferably in the class of phosphatidylcholines, and that the active agent is, where appropriate with an addition of less than 10% w/w relative to the total composition of the preparation, the soluble component, wherein the concentration of the soluble component(s) is typically from 0.01% w/w to 15% w/w, preferably from 0.1% w/w to 10% w/w and particularly preferably from 0.5% w/w to 3% w/w and the total lipid concentration is from 0.005% w/w to 40% w/w, preferably from 0.5% w/w to 15% w/w and particularly preferably from 1% w/w to 10% w/w.

**18.** The use according to any one of claims 1 to 17, **characterized in that** the preparation contains consistency builders such as hydrogels, antioxidants such as probucol, tocopherol, BHT, ascorbic acid, desferroxamine and/or stabilizers such as phenol, cresol, benzyl alcohol or similar substances.

**19.** The use according to any one of claims 1 to 18, **characterized in that** the active substance is a growth modulating substance for living organisms.

**20.** The use according to any one of claims 1 to 18, **characterized in that** the active substance has biocidal properties, in particular insecticidal, pesticidal, herbicidal or fungicidal.

**21.** The use according to any one of claims 1 to 28, **characterized in that** the active substance is an attractant, particularly a pheromone.

**22.** A method of manufacture of a preparation for use in accordance with any one of claims 1 to 21, **characterized in that** at least two amphiphilic components are selected that differ by a factor of at least 10 in their solubility in the suspension medium of the preparation, usually water, and that the content of solubilizing components is less than 0.1mol % relative to the content of these substances, at which the solubilization point of the coated droplets is reached, or else this point cannot be reached in practice in the relevant range, and the amount of the amphiphilic components is set in such a way that the capability of the preparation to permeate through constrictions, amounts to at least 0.01 thousandth of the permeation capacity of water.

**23.** A method according to claim 22, **characterized in that** the amount of amphiphilic components is adjusted such that the ratio of the permeation capacity versus that of water, when the barrier itself is the site of measurement, lies from $10^{-5}$ to 1, preferably from $10^{-4}$ to 1, particularly preferably from $10^{-2}$ to 1.

**24.** A method according to any one of claims 22 and 23, **characterized in that** the stability and permeation capacity are determined by means of filtration, if appropriate under pressure, through a fine-pore filter or through otherwise controlled mechanical fluidization, shearing or fragmentation.

**25.** A method according to any one of claims 22 to 24, **characterized in that** the mixture of substances, for the production of a transfersome®-type of preparation, undergoes filtration, ultrasound, stirring, shaking or other mechanical form of fragmentation.

**26.** A method according to any one of claims 22 to 25, **characterized in that** transfersome®-type droplets forming

the preparation are produced from at least two amphiphilic components of differing polarity, at least one polar liquid and at least one active substance.

27. A method according to any one of claims 22 to 26, **characterized in that** transfersome®-type droplets forming the preparation are produced from at least two amphiphilic components of differing polarity and at least one polar liquid , wherein the amphiphilic component(s) comprises or includes the active substance.

28. A method according to any one of claims 22 to 27, **characterized in that** the amphiphilic components and the hydrophilic substance with the active substance are separately mixed and, where appropriate, placed in solution, the mixtures or solutions then being mixed together and with the addition of particularly mechanical energy the droplets are formed.

29. A method according to any one of claims 22 to 28, **characterized in that** the amphiphilic components are added to a polar solution either as such or dissolved in a physiologically acceptable solvent or solubilizer miscible with polar liquid(s), particularly water.

30. A method according to any one of claims 22 to 29, **characterized in that** the formation of the coated droplets is achieved by stirring and evaporation out of a reverse phase, using an injection or dialysis procedure, using electrical, thermal of mechanical means such as shaking, stirring, homogenizing, ultrasound, trituration, freezing, or thawing, heating or cooling or high- or low-pressure filtration.

31. A method according to any one of claims 22 to 30, **characterized in that** the formation of coated droplets is achieved by filtration and the filter material has a pore size of 0.01 to 0.8 μm, preferably 0.05 to 0.3 μm and particularly preferably 0.08 to 0.15 μm, wherein, if appropriate, a series of several successive filters can be employed.

32. A method according to any one of claims 22 to 31, **characterized in that** the carrier-active agent combination occurs at least partially after formation of the droplets.

33. A method according to any one of claims 22 to 32, **characterized in that** the coated droplets are prepared from a concentrate or lyophilisate shortly before their use.

## Revendications

1. Utilisation de gouttelettes de liquide pouvant rester en suspension dans un milieu liquide, pourvues d'une enveloppe membranaire constituée d'une couche ou de quelques couches d'un vecteur amphiphile, ce vecteur comportant au moins deux composants différents rapportés à leurs propriétés (physico)-chimiques ayant une solubilité différente dans le milieu de suspension de la préparation - normalement de l'eau - d'un facteur d'au moins 10, la concentration d'agents solubilisants étant inférieure à 0,1 en % de mol rapporté à la concentration de ces substances à laquelle le point de solubilisation des gouttelettes enrobées peut être atteint ou bien ne peut pas être atteint, pour la fabrication d'une préparation pour application non invasive ou transport non invasif d'au moins un principe actif, notamment à des fins médicales ou biologiques, dans et à travers des barrières et resserrements telles que ceux de la peau et structures analogues.

2. Utilisation selon la revendication 1, **caractérisée en ce que** les composants amphiphiles sont choisis de façon telle, qu'il ne se produise pas de solubilisation indépendante de la concentration.

3. Utilisation selon l'une des revendications 1 et 2, **caractérisée en ce que** la solubilité, en particulier l'hydrosolubilité du (des) composant(s) plus soluble(s) soit comprise entre au moins $10^{-3}$ et $10^{-6}$ M.

4. Utilisation selon l'une des revendications 1 à 3, **caractérisée en ce que** la différence de solubilité entre le(s) composant(s) soluble(s) et le(s) composant(s) moins soluble(s) soit comprise entre 10 et $10^7$, de préférence entre $10^2$ et $10^6$ et plus particulièrement, entre $10^3$ et $10^5$.

5. Utilisation selon l'une des revendications 1 à 4, **caractérisée en ce que** la capacité de perméation de la préparation dans les resserrements est de 0,01 pour mille, de préférence de 1 pour mille de la perméabilité des petites molécules pénétrant pour l'essentiel librement.

6. Utilisation selon l'une des revendications 1 à 5, **caractérisée en ce que** le rapport de la capacité de perméation par rapport aux particules de référence P ($_{Tansf}$/$P_{(Refer)}$), les particules de référence, par exemple l'eau, étant beaucoup plus petites que les resserrements de la barrière lorsque la barrière constitue elle-même l'emplacement d'évaluation, est comprise entre $10^{-5}$ et 1, de préférence entre $10^{-4}$ et 1 et plus particulièrement entre $10^{-2}$ et 1.

7. Utilisation selon l'une des revendications 1 à 6, **caractérisée en ce que** la préparation comporte une concentration d'au moins deux composants amphiphiles de solubilité différente pour former un vecteur et/ou une enveloppe membranaire entourant une quantité de gouttelettes de liquide hydrophile, le principe actif se trouvant dans le vecteur ou sur l'enveloppe membranaire, et/ou dans le liquide hydrophile.

8. Utilisation selon l'une des revendications 1 à 7, **caractérisée en ce que** le rayon de la vésicule des gouttes contenues dans l'enveloppe est compris entre environ 25 et environ 500 nm, de préférence entre environ 50 et environ 200 nm, plus particulièrement entre environ 80 et environ 180 nm.

9. Utilisation selon l'une des revendications 1 à 8, **caractérisée en ce que** l'enveloppe est une bicouche.

10. Utilisation selon l'une des revendications 1 à 9, **caractérisée en ce que** le(s) composant(s) amphiphile(s) comporte (nt) des lipides physiologiquement tolérés de polarités différentes et/ou un tel ou de tels principe(s) actif(s).

11. Utilisation selon l'une des revendications 1 à 10, **caractérisée en ce que** la substance amphiphile est un lipide ou un lipoïde d'origine biologique ou un lipide synthétique équivalent ou un dérivé de ce lipide, en particulier un dérivé de diacyle- ou de dialkyle-glycérophosphoéthanolamine-azopolyéthylène, de la didécanoylphosphatidylcholine, de la diacylphospho-oligomaltobionamide, un glycéride, un glycérophospholipide, un lipide isopropénoïde, un sphingolipide, un stéroïde, une stérine ou stérol, un lipide contenant du soufre ou un hydrate de carbone, ou bien un autre lipide formant des structures stables, par exemple des bicouches, de préférence un acide gras liquide semi-protoné, en particulier une phosphatidylcholine, une phosphatidyléthanolamine, un phosphatidylglycérol, un phosphatidylinositol, un acide phosphatidique, une phosphatidylsérine, une sphingomyéline ou un sphingophospholipide, un glycosphingolipide (par exemple, cérébroside, céramide polyhexoside, sulfatide, sphingoplasmalogène), un ganglioside ou autre glycolipide, ou un lipide synthétique, de préférence un dioleoyl-, dilinolyl-, dilinoényl-, dilinoloyl-, dilinolinayl-, diarachinoyl-, dilauroyl-, dimyristoyl-, dilalmitoyl-, distéaroylphospholipide ou un dérivé de dialkyle ou de sphingosine équivalent, un glycolipide ou autres lipides acyle ou alkyle à chaîne identique ou combinée.

12. Utilisation selon l'une des revendications 1 à 11, **caractérisée en ce que** le composant moins soluble contient un lipide synthétique, de préférence un myristeoleoyl-, pétrosélinyl-, pétroselaidyl-, un oléoyl-, élaidyl-, *cis*- ou *trans*-vaccenoyl-, linolyl-, linolényl-, linolaidyl-, octadécatétraénoyl-, gondoyl-, éicosaenoyl-, éicosadiénoyl-, éicosatriénoyl-, arachidoyl-, *cis*- ou *trans*-docosaenoyl-, docosadiénoyl-, docosatrienoyl-, docosatetraénoyl-, caproyl-, lauroyl-, tridécanoyl-, myristoyl-, pentadecanoyl-, palmytoyl-, heptadecanoyl-, stéaroyl- ou nonadécanoylglycérophospholipide, ou un dérivé équivalent à chaîne ramifiée ou un sphingodérivé équivalent, un glycolipide ou autres lipides acyle ou alkyle ; on dérive un (des) composant(s) amphiphile(s) plus soluble(s) de l'un des composants moins solubles énumérés ci-dessus, et pour accroître la solubilité, on le dérive avec un substituant butanoyle, pentanoyle, hexanoyle, heptanoyle, octanoyle, nonanoyle, décanoyle, dodécan- ou undecanoyle équivalent de ceux-ci, mono- ou polyinsaturé ou à chaîne ramifiée, ou avec plusieurs substituants choisis indépendamment les uns des autres, et/ou on effectue une dérivatisation et/ou une substitution, une complexation et/ou une association avec une autre substance appropriée pour améliorer la solubilité.

13. Utilisation selon l'une des revendications 1 à 12, **caractérisée en ce que** la concentration de substance amphiphile destinée à l'application sur les peaux humaine et animale est comprise entre 0,01 et 40% en poids de la préparation, de préférence entre 0,1 et 15% en poids et plus particulièrement entre 1 et 10% en poids.

14. Utilisation selon l'une des revendications 1 à 13, **caractérisée en ce que** la concentration totale de substance amphiphile destinée à l'application sur les plantes est comprise entre 0,000001 et 10% en poids de la préparation, de préférence entre 0,001 et 1% en poids et plus particulièrement entre 0,01 et 0,1% en poids.

15. Utilisation selon l'une des revendications 1 à 14, **caractérisée en ce que** la préparation renferme comme principe actif un adrénocorticostatique, un β-adrénolytique, un androgène ou un anti-androgène, un antiparasitaire, un anabolisant, un anesthésique ou un analgésique, un analeptique, un anti-allergique, un anti-arythmique, un anti-artériosclérotique, un anti-asthmatique et/ou un bronchospasmolytique, un antibiotique, un antidépresseur et/ou

un antipsychotique, un antidiabétique, un antidote, un anti-émétique, un anti-épileptique, un antifibrinolytique, un anticonvulsif, un anticholinergique, un enzyme, un co-enzyme ou un inhibiteur équivalent, un antihistaminique, un antihypertonique, un inhibiteur d'activité biologique, un antihypotonique, un anticoagulant, un antifongique, un antimyasthénique, un agent thérapeutique contre la maladie de Parkinson ou d'Alzheimer, un antiphlogistique, un antipyrétique, un antirhumatismal, un antiseptique, un analeptique ou stimulant respiratoire, un broncolytique, un cardiotonique, un agent chimiothérapeutique, un coronarodilatateur, un cytostatique, un diurétique, un bloqueur ganglionnaire, un glucocorticoïde, un anti-grippal, un hémostatique, un hypnotique, de l'immunoglobuline ou un fragment de celle-ci, ou un autre anticorps ou équivalent, un hydrate de carbone (dérivé) bioactif, un contraceptif, un antimigraineux, un corticoïde minéral, un antagoniste de la morphine, un myorelaxant, un narcotique, un agent thérapeutique pour les nerfs ou le SNC, un nucléotide ou un polynucléotide, un neuroleptique, un neurotransmetteur ou un antagoniste équivalent, un peptide (dérivé), un agent ophtalmique, un (para)-sympaticométique ou un (para)-sympathicolytique, une protéine (dérivé), un antipsoriasis/névrodermite, un mydriatique, un psychostimulant, un antirhinitique, un somnifère ou ses antagonistes, un sédatif, un spasmolytique, un antituberculeux, un agent urologique, un vasoconstricteur ou dilatateur, un anti-viral ou un cicatrisant ou plusieurs de ces agents, en particulier du diclofénac ou de l'ibuprofène.

**16.** Utilisation selon l'une des revendications 1 à 15, **caractérisée en ce que** le principe actif est un anti-inflammatoire non stéroïdien, par exemple du diclofénac, de l'ibuprofène, un sel de lithium, de sodium, de potassium, de césium, de rubidium, d'ammonium, d'ammonium de monométhyle, de diméthyle, de triéthyle ou d'éthyle.

**17.** Utilisation selon l'une des revendications 1 à 16, **caractérisée en ce que** le composant moins polaire contient un lipide physiologiquement toléré, de préférence de la classe des phospholipides, plus particulièrement de la classe des phosphatidylcholines, et **en ce que** le principe actif est le composant le plus soluble, le cas échéant avec addition de moins de 10% en poids, rapporté à la composition globale de la préparation, d'un autre composant soluble, la concentration de composant(s) soluble(e) étant normalement comprise entre 0,01% en poids et 15% en poids, et plus particulièrement entre 0,5% en poids et 3% en poids, et la concentration de lipides totale, entre 0,005% en poids et 40% en poids, de préférence entre 0,5% en poids et 15% en poids et plus particulièrement, entre 1 % en poids et 10% en poids.

**18.** Utilisation selon l'une des revendications 1 à 17, **caractérisée en ce que** la préparation contient des épaississants tels que des hydrogels, des anti-oxydants tels que le probucol, le tocophérol, le BHT, l'acide ascorbique, la desferroxamine et/ou des stabilisateurs tels que le phénol, le crésol, l'alcool benzylique et substances analogues.

**19.** Utilisation selon l'une des revendications 1 à 18, **caractérisée en ce que** le principe actif est un modulateur de croissance des organismes vivants.

**20.** Utilisation selon l'une des revendications 1 à 18, **caractérisée en ce que** le principe actif possède des propriétés biocides, et est notamment un insecticide, un pesticide, un herbicide ou un fongicide.

**21.** Utilisation selon l'une des revendications 1 à 18, **caractérisée en ce que** le principe actif est un attractif, notamment une phéromone.

**22.** Procédé pour la fabrication d'une préparation destinée à être utilisée selon l'une des revendications 1 à 21, **caractérisé en ce qu'** il se différencie, dans sa solubilité dans le milieu de suspension de la préparation - normalement de l'eau - par un facteur d'au moins 10, et que la concentration d'agents solubilisants est inférieure à 0,1% en mol, rapporté à la concentration de ces substances à laquelle le point de solubilisation des gouttelettes enrobées est atteint ou bien ne peut pas être atteint dans une plage intéressante en pratique, et en ce que la concentration de composants amphiphiles est ajustée de façon telle, que la capacité de perméation de la préparation dans les resserrements soit au moins de 0,01 millième de la capacité de perméation de l'eau.

**23.** Procédé selon la revendication 22, **caractérisé en ce que** l'on ajuste la concentration de composants amphiphiles de façon telle, que le rapport de capacité de perméation par rapport à l'eau soit, lorsque la barrière constitue elle-même l'emplacement d'évaluation, compris entre $10^{-5}$ et 1, de préférence entre $10^{-4}$ et 1, et plus particulièrement entre $10^{-2}$ et 1.

**24.** Procédé selon l'une des revendications 22 et 23, **caractérisé en ce que** l'on évalue la stabilité et la capacité de perméation par filtration, le cas échéant sous pression, au moyen d'un filtre à pores fins ou autre méthode de fluidisation, cisaillement ou fragmentation mécanique contrôlée.

**25.** Procédé selon l'une des revendications 22 à 24, **caractérisé en ce que** le mélange de substances est, pour la production d'une préparation de type Transfersom®, soumis à une filtration, à un traitement aux ultrasons, à un mélange, à une agitation ou à d'autres actions mécaniques exerçant une action de désagrégation.

**26.** Procédé selon l'une des revendications 22 à 25, **caractérisé en ce que** l'on fabrique à partir d'au moins deux composants amphiphiles de polarité différente, d'au moins un liquide polaire et d'au moins un principe actif, des gouttelettes de type Transfersom®, qui constituent la préparation.

**27.** Procédé selon l'une des revendications 22 à 26, **caractérisé en ce que** l'on fabrique à partir d'au moins deux composants amphiphiles de polarité différente et d'au moins un liquide polaire, des gouttelettes type Transfersom® qui constituent la préparation dans laquelle le(s) composant(s) amphiphiles contient(nnent) ou renferme(nt)le principe actif.

**28.** Procédé selon l'une des revendications 22 à 27, **caractérisé en ce que** l'on mélange séparément les composants amphiphiles et la substance hydrophile avec le principe actif, que le cas échéant, on les met dans une solution, le mélange et les solutions étant ensuite réunis en un mélange, et que l'on provoque la formation des gouttelettes dans celui-ci notamment par apport d'énergie mécanique.

**29.** Procédé selon l'une des revendications 22 à 28, **caractérisé en ce que** les composants amphiphiles sont ajoutés à une solution polaire, soit tels quels soit dilués dans un solvant physiologiquement toléré ou un agent solubilisant miscible avec un (des) liquide(s) polaire(s), en particulier de l'eau.

**30.** Procédé selon l'une des revendications 22 à 29, **caractérisé en ce que** la formation des gouttelettes enrobées est provoquée par délayage, par évaporation à partir d'une phase inverse, par injection ou par dialyse, par contrainte électrique, thermique ou mécanique telle qu'agitation, secousse, homogénéisation, traitement aux ultrasons, trituration, congélation et décongélation, chauffage ou refroidissement, ou filtration sous haute ou basse pression.

**31.** Procédé selon l'une des revendications 22 à 30, **caractérisé en ce que** la formation des gouttelettes est provoquée par filtration et que le matériau de filtration a une grosseur de pores comprise entre 0,01 et 0,8 μm, de préférence entre 0,05 et 0,3 μm et plus particulièrement, entre 0,08 et 0,15 μm, une série de plusieurs filtres successifs, pouvant, le cas échéant, être utilisée.

**32.** Procédé selon l'une des revendications 22 à 31, **caractérisé en ce que** l'association vecteur-principe actif s'effectue partiellement après la formation des gouttelettes.

**33.** Procédé selon l'une des revendications 22 à 32, **caractérisé en ce que** les gouttelettes enrobées sont préparées à partir d'un concentrat ou d'un lyophilisat peu avant d'être utilisées.

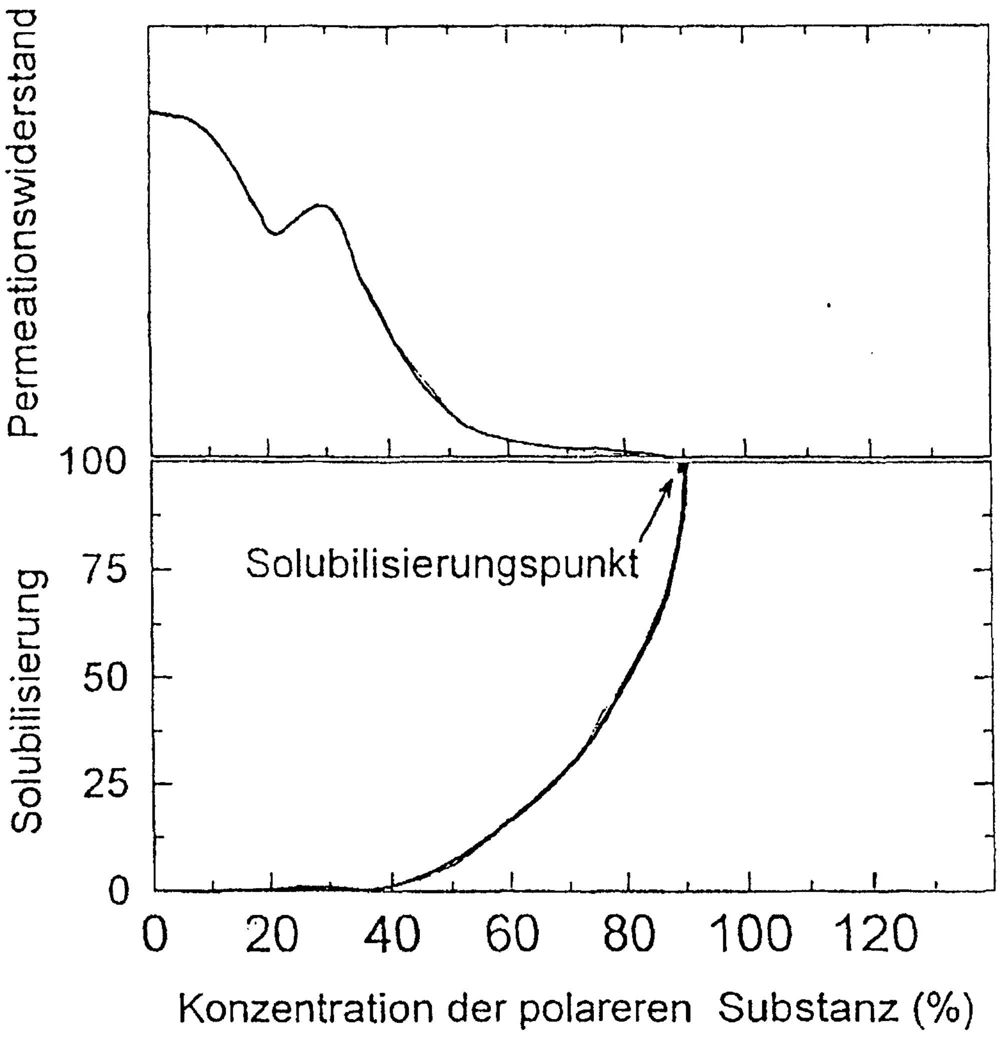
Permeationswiderstand
Solubilisierung
100
75
50
25
0
Solubilisierungspunkt
0 20 40 60 80 100 120
Konzentration der polareren Substanz (%)

FIGUR 1

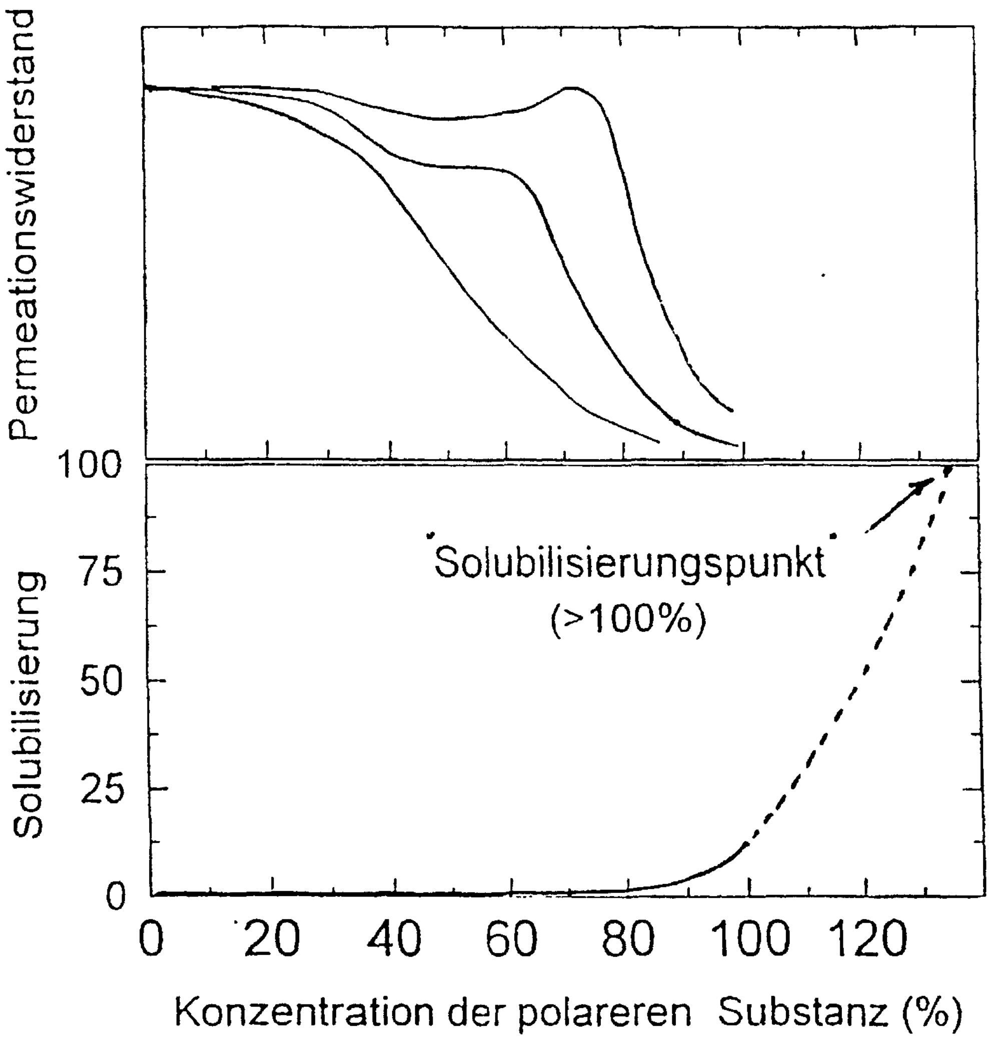
Permeationswiderstand
Solubilisierung
100
75
50
25
0
Solubilisierungspunkt
(>100%)
0
20
40
60
80
100
120
Konzentration der polareren Substanz (%)

FIGUR 2

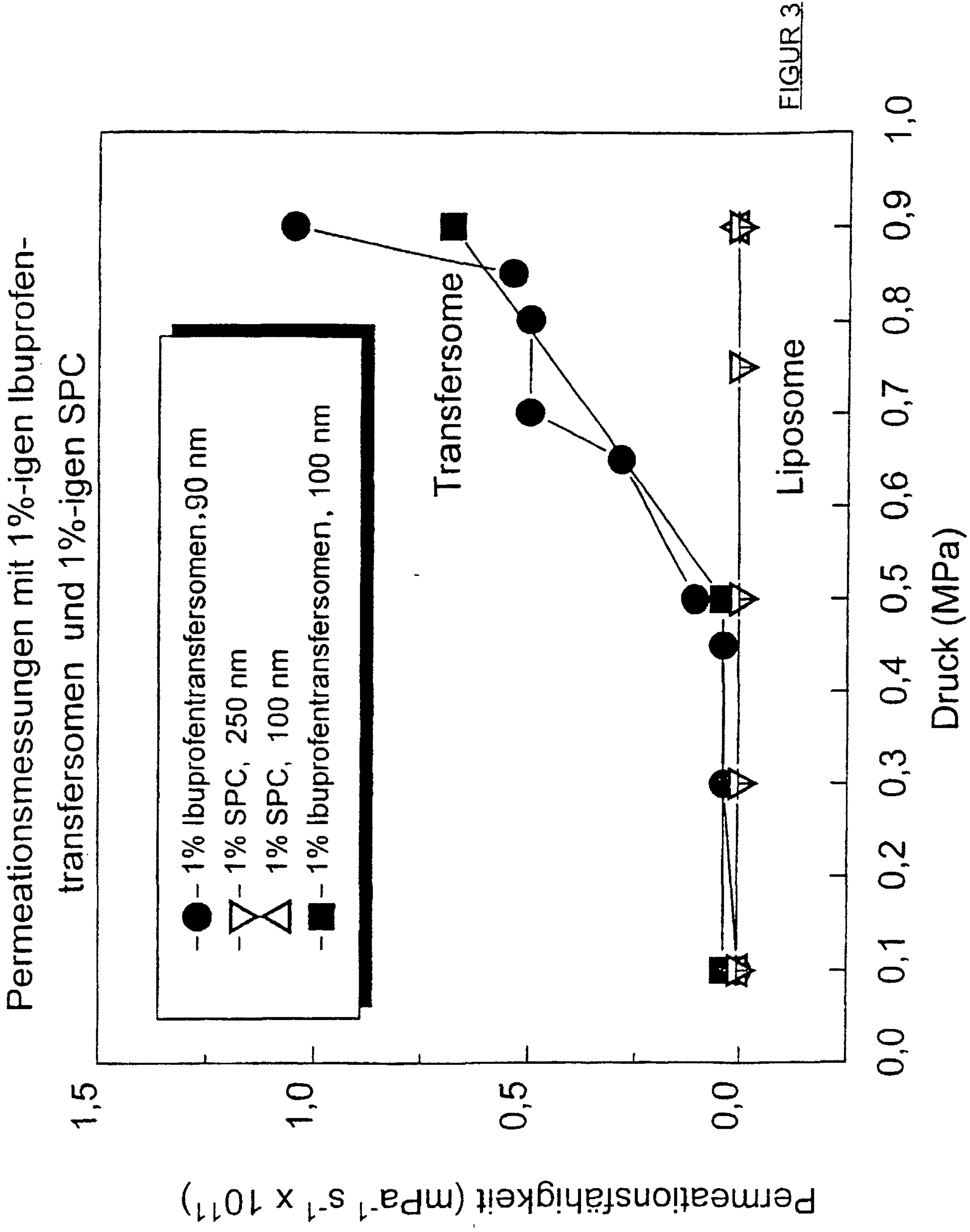
Permeationsmessungen mit 1%-igen Ibuprofen-
transfersomen und 1%-igen SPC
1% Ibuprofentransfersomen, 90 nm
1% SPC, 250 nm
1% SPC, 100 nm
1% Ibuprofentransfersomen, 100 nm
Transfersome
Liposome
Permeationsfähigkeit (mPa$^{-1}$ s$^{-1}$ x $10^{11}$)
1,5
1,0
0,5
0,0
Druck (MPa)
0,0
0,1
0,2
0,3
0,4
0,5
0,6
0,7
0,8
0,9
1,0

FIGUR 3

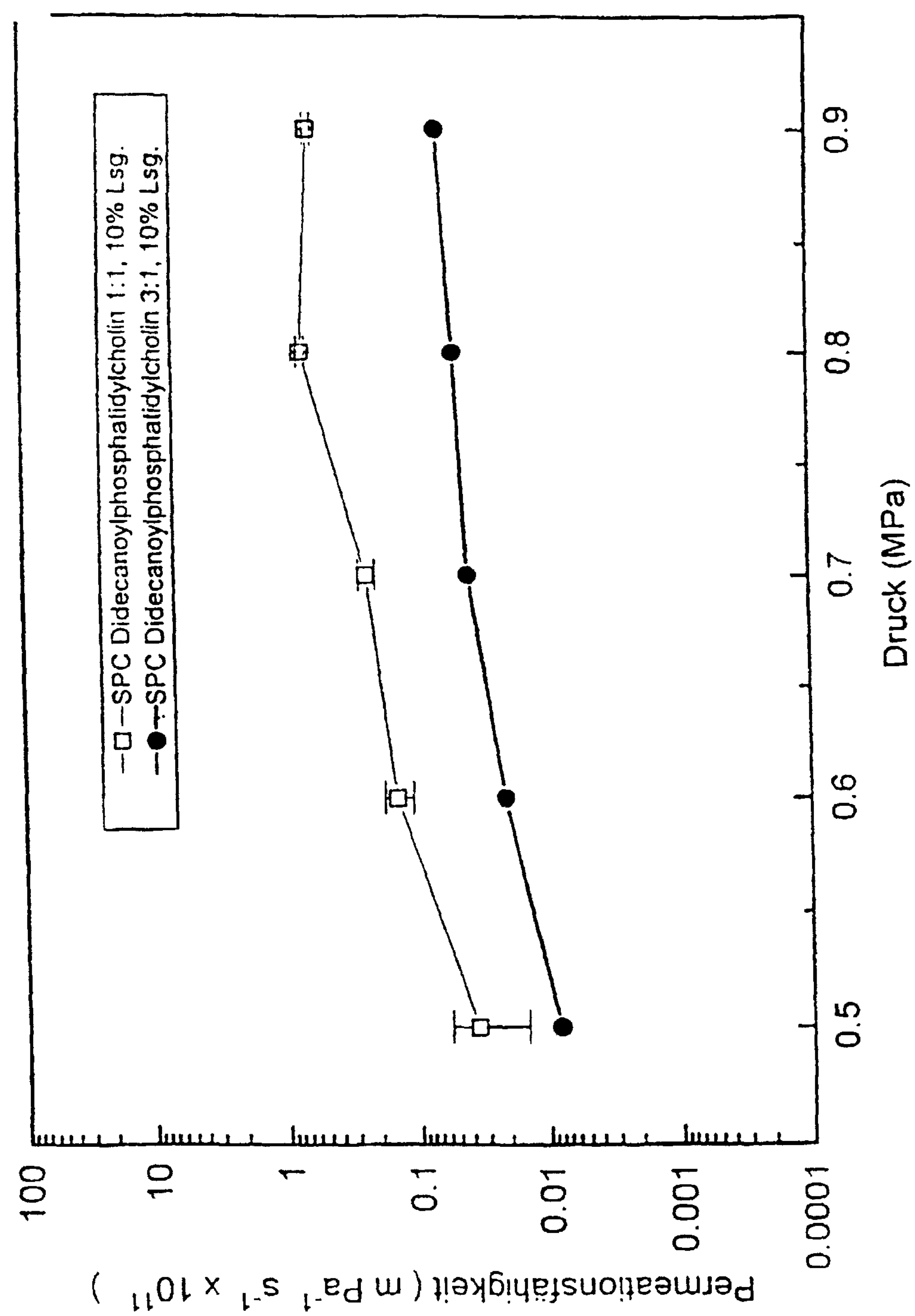
SPC Didecanoylphosphatidylcholin 1:1, 10% Lsg.
SPC Didecanoylphosphatidylcholin 3:1, 10% Lsg.
Permeationsfähigkeit ( m Pa$^{-1}$ s$^{-1}$ x 10$^{11}$ )
100
10
1
0.1
0.01
0.001
0.0001
0.5
0.6
0.7
0.8
0.9
Druck (MPa)

FIGUR 4

Trübungsmessung per Auge von declofenachaltigen Transfersomen mit unterschiedlichen Verhältnissen von Lipid zu Detergens

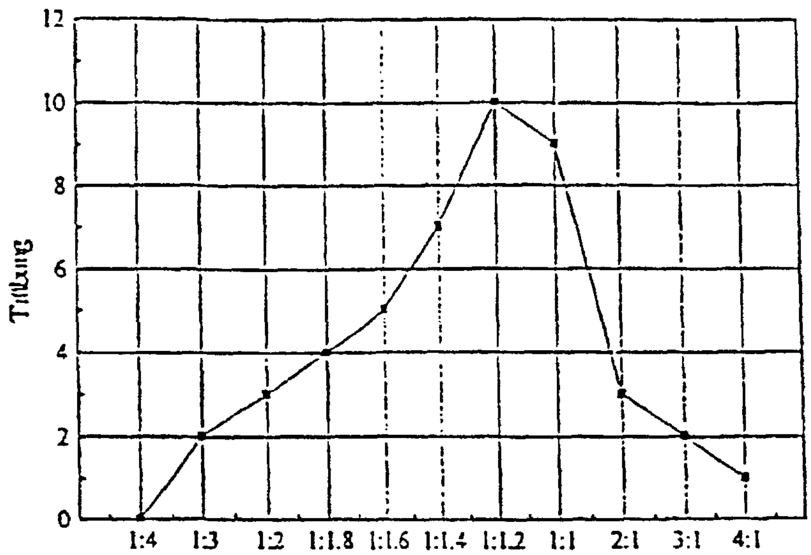


Verhältnis Lipid (SPC): Detergens (Diclofenac)

FIGUR 5

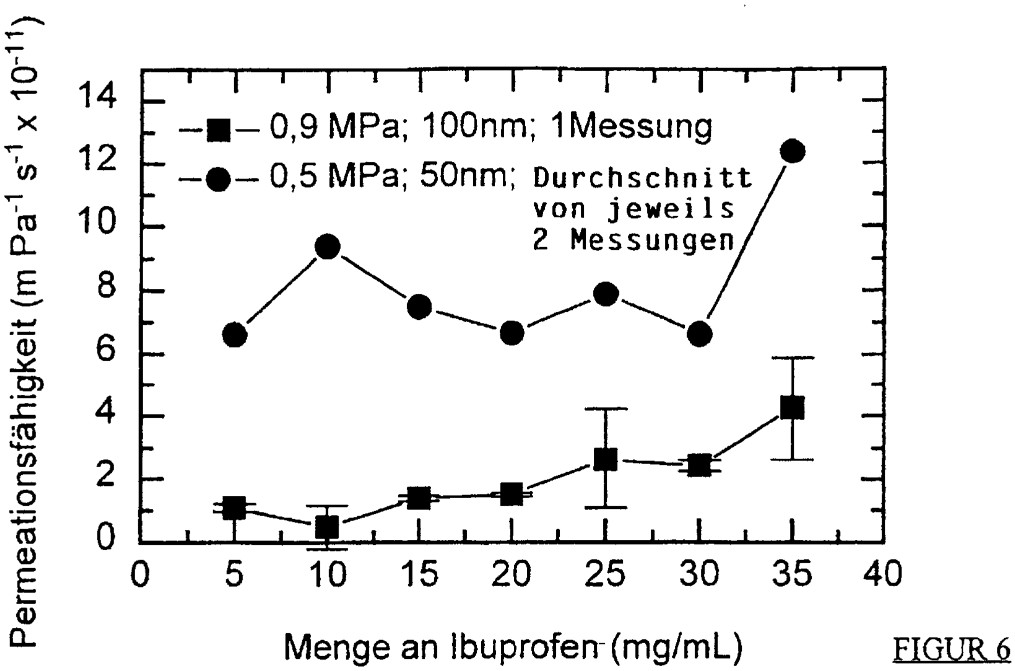


FIGUR 6

Die Teilchengröße wird durch dynamische Lichtstreuung bestimmt.

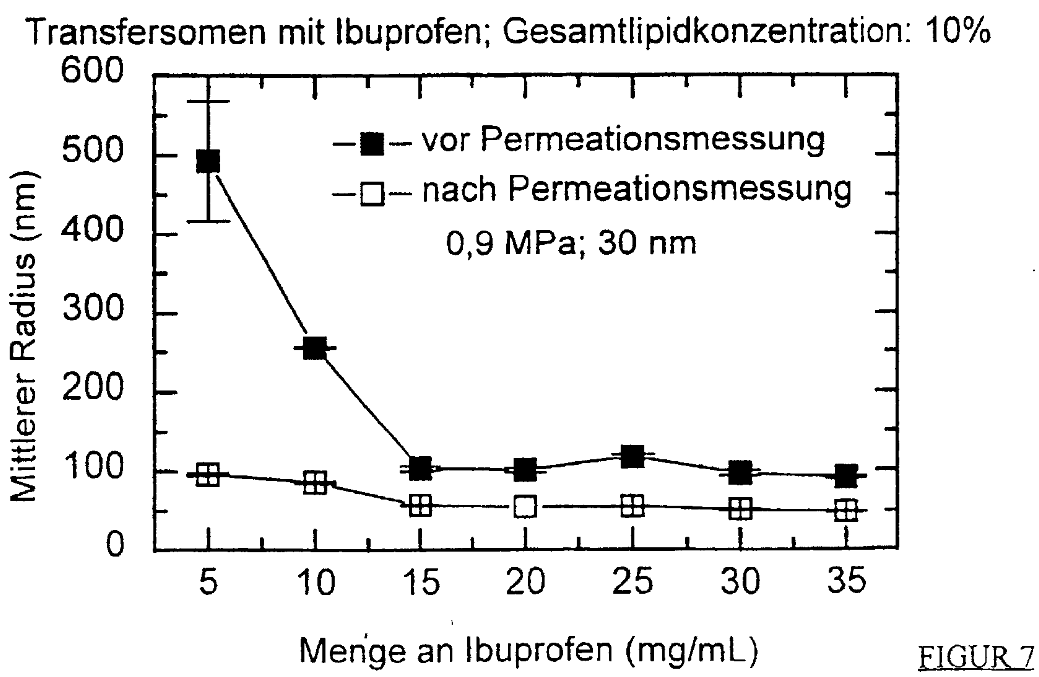


FIGUR 7

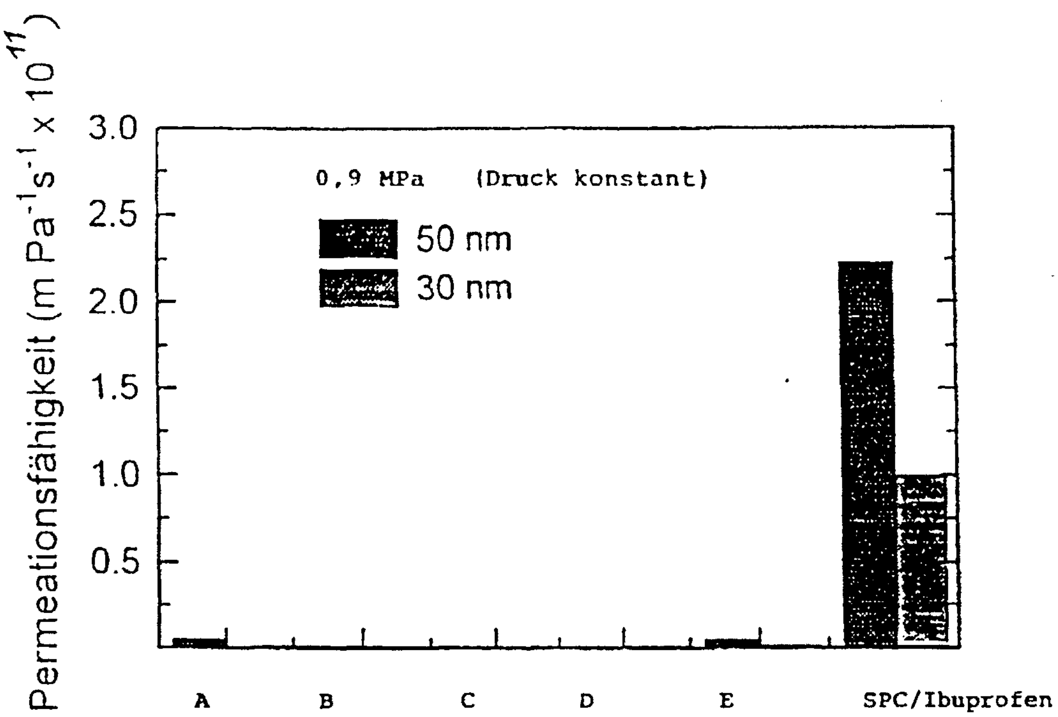

Permeationsfähigkeit (m Pa$^{-1}$s$^{-1}$ x 10$^{11}$)
3.0
2.5
2.0
1.5
1.0
0.5
0,9 MPa (Druck konstant)
50 nm
30 nm
A
B
C
D
E
SPC/Ibuprofen


FIGUR 8